(19)

Europäisches
Patentamt
European
Patent Office
Office européen
des brevets

(11) **EP 4 563 346 A1**

(12) **EUROPEAN PATENT APPLICATION**
published in accordance with Art. 153(4) EPC

(43) Date of publication:
**04.06.2025 Bulletin 2025/23**

(21) Application number: **23846495.2**

(22) Date of filing: **25.07.2023**

(51) International Patent Classification (IPC):
**B32B 27/12** (2006.01)       **A61L 15/26** (2006.01)
**A61L 15/28** (2006.01)       **A61L 15/42** (2006.01)
**A61P 17/02** (2006.01)       **B32B 5/02** (2006.01)
**B32B 27/36** (2006.01)

(52) Cooperative Patent Classification (CPC):
**A61L 15/26; A61L 15/28; A61L 15/42; A61P 17/02;**
**B32B 5/02; B32B 27/12; B32B 27/36**

(86) International application number:
**PCT/JP2023/027115**

(87) International publication number:
**WO 2024/024765 (01.02.2024 Gazette 2024/05)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **25.07.2022 JP 2022117800**

(71) Applicant: **Toray Industries, Inc.**
**Tokyo 103-8666 (JP)**

(72) Inventors:
• **HOCHI, Motonori**
  **Otsu-shi, Shiga 520-2141 (JP)**
• **HARA, Yoshitake**
  **Otsu-shi, Shiga 520-2141 (JP)**

(74) Representative: **Kador & Partner Part mbB**
**Corneliusstraße 15**
**80469 München (DE)**

(54) **MULTILAYER OBJECT**

(57)     An object of the present invention is to provide a laminate that has excellent handleability when attached to an adherend, has excellent durability when in a dry state, and has not difference in characteristics between both of the outermost faces. The present invention provides a laminate including: an adhesion-preventing layer (A) composed of a polylactic acid resin and having a thickness of 10 to 500 nm: a water-soluble film (B) composed of a water-soluble resin and having a thickness of 1 to 18 μm; and a water-soluble nonwoven fabric (C) having a thickness of 80 to 550 μm; in which the layer, the film, and the fabric are laminated in the order A/B/C/B/A; and wherein the laminate has a specified cross-sectional density and specified structural characteristics so that the laminate can achieve excellent handleability when attached to an adherend, has excellent durability in a dry state, and has no difference between both of the faces.

Fig.3

## Description

Technical Field

The present invention relates to a laminate.

Background Art

Surgical operations typified by abdominal surgery, orthopedic surgery, neurosurgery, and the like involve postsurgical complications, one of which is a problem of organ-to-organ adhesion. This is because, when tissues damaged by postsurgical drying and oxidation are sutured in a closed manner, a phenomenon of adhesion, i.e., coaptation can occur in the self-healing process due to wound healing between the organ tissues that should originally not be coapted. Such adhesion is said to be caused with a high probability in a surgical operation, and can lead to pain or a complication that induces a serious pathological condition such as ileus or infertility.

Adhesion, once caused, is difficult to treat with medication. In some cases, ileus is caused by adhesion several years after surgery. It is believed that adhesion can be treated only through adhesiotomy in which an area of adhesion is ablated through a surgical operation. In the surgical operation, it is important to prevent adhesion and prevent delay of healing.

A conventionally known material effective for such prevention of adhesion and such prevention of delay of healing is an adhesion-preventing material in which gelatin, collagen, or the like expected to be bioabsorbable is used to separate an organ tissue physically. In addition, an adhesion-preventing material needs to indwell in the body after surgery, and is preferably decomposed after a surgical site is healed. Hence, the material needs to be low in the risk of infection, and high in safety to organisms and in decomposability and absorbability in organisms.

There is another known adhesion-preventing material in which a plurality of layers having a specific function are laminated to give a plurality of functions to the material. For example, a laminate composed of a fibrous structure containing a water-soluble polymer and of a polylactic acid resin having a thickness in nanometers is disclosed; in which the laminate has excellent handleability owing to the thickness of the fibrous structure when attached to an adherend; in which, after the laminate is attached to the adherend, water is allowed to stick to the fibrous structure, enabling only the layer containing the polylactic acid resin to remain on a tissue; and in which the layer containing the water-soluble polymer is provided between the polylactic acid resin and the fibrous structure, thereby enhancing adhesiveness between the polylactic acid resin and the fibrous structure (Patent Document 1).

In addition, Patent Document 1 discloses a laminate in which the polylactic acid resin having a thickness in nanometers and the fibrous structure containing a water-soluble polymer are laminated, between which a layer containing a water-soluble polymer is sandwiched, thus increasing the adherence and enhancing the handleability. However, the fibrous structure containing a water-soluble polymer is exposed on the outermost layer, and thus, there is not only a possibility that the solubility of the laminate attached to an adherend has irregularity, for example, owing to unevenness in the areal weight of the fibrous structure but also a possibility that the laminate results in having poorer handleability when attached to an adherend, because the laminate is easily dissolved if wetted by mistake when attached to the adherend. In a method of improving these problems, a laminate is disclosed, the laminate having a constitution in which a film containing a water-soluble polymer is laminated on the surface of a fibrous structure, whereby the laminate not only has water resistance when handled but also has water-solubility after being attached to an organ. (Patent Document 2)

In addition, a raw material to be used as an adhesion-preventing material is often a material having biocompatibility and biodegradability, and is commonly protected by a gas-barrier package less prone to be permeated by oxygen or a moisture-proof package less prone to be permeated by water, from the viewpoint of long-term storage stability. In particular, to lower the influence of moisture-induced deterioration of the material, countermeasures such as enclosing a drying agent in the packaging material to keep low humidity therein for a long time are taken in some cases.

Prior Art Documents

Patent Documents

Patent Document 1: JP 6119863 B
Patent Document 2: JP 7077621 B

Summary of Invention

Problems Which the Invention Tries to Solve

Patent Documents 1 and 2 disclose the material, thickness, and fine structure (film and fiber) of a layer to be laminated and the number of the layers, as such information is necessary to enhance the handleability of the laminate being attached to an organ. However, they have not disclosed the durability of the laminate that is under a dry environment and in a dry state, for example, while the laminate is packaged in a packaging material, or immediately after the packaging material is opened.

In view of this, an object of the present invention is to provide a laminate that has excellent handleability when attached to an adherend, and has excellent durability when in a dry state.

Means for Solving the Problems

The present inventors have made studies vigorously to solve the above-described problems, and have consequently come to discover the following inventions (1) to (3).

(1) A laminate comprising: an adhesion-preventing layer (A) composed of a polylactic acid resin and having a thickness of 10 to 500 nm; a water-soluble film (B) composed of a water-soluble resin and having a thickness of 1 to 18 $\mu$m; and a water-soluble nonwoven fabric (C) composed of a water-soluble resin and having a thickness of 80 to 550 $\mu$m; wherein the adhesion-preventing layer (A), the water-soluble film (B), and the water-soluble nonwoven fabric (C) are laminated in the order (A)/(B)/(C)/(B)/(A); wherein the laminate has a cross section of which scatter diagram satisfies the following Formula 1:

$$0.90 \le I_{10-45} / I_{max} \le 0.99 \ldots \text{Formula 1}$$

(wherein in Formula 1, $I_{10-45}$ represents an average intensity in the angles of from 10 to 45 degrees, and $I_{max}$ represents the maximum average intensity in 0 to 180 degrees);
the scatter diagram being obtained by: observing the cross section under a scanning electron microscope at a magnification of 500 times to obtain a digital image; cutting an image (X) of 720 × 720 pixels out of the digital image; binarizing the image (X) with a threshold by a discriminant analysis method to obtain an image (Y), wherein at the threshold the separation metrics is the maximum in the method; fast-Fourier-transforming the image (Y) to obtain a power spectrum intensity; converting the power spectrum intensity into decibels; and then integrating the converted intensity at 5-degree angular intervals from 0 to 180 degrees on the spectrum to obtain the scatter diagram, wherein in the spectrum the central part is assumed as the origin, the positive x axis represents zero degree, and the counterclockwise direction represents a positive angle, and wherein in the scatter diagram the ordinate is an average intensity (I), and the abscissa is an angle.

(2) The laminate according to (1),
wherein the image (Y) is obtained by:

observing the cross section under the scanning electron microscope at a magnification of 500 times to obtain the digital image;
cutting the image (X) of 720 × 720 pixels out of the digital image; and
binarizing the image (X) with the threshold by the discriminant analysis method to obtain the image (Y), wherein at the threshold the separation metrics is the maximum in the method; and wherein the image (Y) satisfies the following Formula 2:

$$0.30 \le a / \{a + b\} \le 0.45 \ldots \text{Formula 2}$$

(wherein in Formula 2, "a" represents a photographed area of the portion including the adhesion-preventing layer (A), the water-soluble film (B), and the water-soluble nonwoven fabric (C), and "b" represents a photographed area of the vacant portion surrounded by the adhesion-preventing layer (A), the water-soluble film (B), and the water-soluble nonwoven fabric (C)).

(3) The laminate according to (1) or (2), wherein the water-soluble resin is pullulan.

Effects of Invention

The laminate according to the present invention has a cross section allowed to have a structural pattern having a specific parameter, thus making it possible to inhibit the laminate as a whole in a dry state from being embrittled by a change in the mechanical strength of the water-soluble film and the water-soluble nonwoven fabric.

Brief Description of Drawings

FIG. 1 is one example of a schematic diagram of a cross section of a laminate according to the present invention.
FIG. 2 is one example of an image of a laminate according to the present invention, as photographed under a scanning electron microscope.
FIG. 3 is one example of an image of a laminate according to the present invention, before being Fourier-transformed.
FIG. 4 is one example of the image in FIG. 3, after being Fourier-transformed.
FIG. 5 is one example of the position of intensity I in a laminate according to the present invention and one example of a scatter diagram.
FIG. 6 is a schematic diagram illustrating a method for a durability test on a laminate according to the present invention.

Mode for Carrying out the Invention

A laminate according to the present invention will be described in detail below with reference to embodiments.
A "film", a "membrane", a "sheet", and a "layer" as used herein mean a planar structure having a two-dimensional expanse. In addition, a "laminate" in the present invention refers to a sheet-like construct composed of a plurality of layers, in which construct a monolayer or multilayer membrane and film and a monolayer or multilayer nonwoven fabric are laminated on each other. In addition, a "cross section of a laminate" as used herein refers to a cut face generated by cutting a sheet-like laminate perpendicularly to the plane of the sheet, and is used to mean a face observed in the direction perpendicular to the direction of thickness.
A laminate according to the present invention has a structure in which the below-described adhesion-preventing layer (A), water-soluble film (B), and water-soluble nonwoven fabric (C) are laminated in a specific order.

<Adhesion-preventing Layer (A)>

The adhesion-preventing layer (A) is composed of a polylactic acid resin, and the thickness of the layer is 10 nm to 500 nm from the viewpoint of shape-conformability to an adherend, and is more preferably 10 nm to 300 nm, still more preferably 50 nm to 200 nm. The layer having a thickness of less than 10 nm may cause the laminate to have insufficient water resistance, or make it difficult for the adhesion-preventing layer to retain its shape. The layer having a thickness of more than 500 nm may cause the laminate to generate wrinkles when attached to an adherend, to become prone to cause a slip between an intended position of attachment and the adhesion-preventing layer, or to take time to decompose in an organism.
The adhesion-preventing layer (A) may contain an impact modifier in an amount of 2 mass% or more and 20 mass% or less with respect to 100 mass% of the whole polylactic acid resin layer in order to possess enhanced mechanical strength. The amount is preferably 2.5 mass% or more and 15 mass% or less. The higher the amount of the impact modifier, the more enhanced the effect of improving the impact resistance. In some cases, however, containing the impact modifier at more than 20 mass% does not enhance the mechanical strength significantly.
To the extent that the effects of the present invention are not impaired, an additive of any kind may be contained at 30 mass% or less with respect to 100 mass% of the whole adhesion-preventing layer (A). Examples of the additive that is usable include antioxidants, weathering agents, heat stabilizers, lubricants, nucleating agents, ultraviolet absorbers, and colorants. The amount of the additive is not particularly limited to any lower limit, and may be 0 mass% with respect to 100 mass% of the whole adhesion-preventing layer (A).
In addition, a bioabsorbable material composed of gelatin, collagen, hyaluronic acid, chitosan, synthetic polypeptide, and the like may be added as the another additive to adjust the rate of biodegradability suitably.
In addition, to the extent that the effects of the present invention are not impaired, organic or inorganic particles may be contained at 20 mass% or less with respect to 100 mass% of the whole adhesion-preventing layer (A). Examples of the inorganic or organic particles include: particles of calcium carbonate, titanium oxide, silicon oxide, calcium fluoride, lithium fluoride, alumina, barium sulfate, zirconia, calcium phosphate, cross-linked polystyrene and metal nanoparticles. The amount of the inorganic or organic particles is not particularly limited to any lower limit, and may be 0 mass% with respect to 100 mass% of the whole adhesion-preventing layer (A).

An adhesion-preventing layer (A) according to the present invention is transparent, and thus, the face of attachment is unnoticeable. The laminate can not only be used in surgery but also be attached to the skin, and be used as an adhesive plaster.

Furthermore, the adhesion-preventing layer (A) can be used as a base material to carry various kinds of drug, and release the drug in a sustained manner, and can also be used as a drug delivery system.

<Water-soluble Film (B)>

A water-soluble film (B) in the present invention may be constituted by one kind of water-soluble resin, or may be constituted by two or more kinds of water-soluble resins.

The thickness of the water-soluble film (B) is preferably 1 $\mu$m to 18 $\mu$m, more preferably 2 $\mu$m to 10 $\mu$m, from the viewpoints of the adhesive strength to the water-soluble nonwoven fabric (C), the handleability of the laminate, and the shape-conformability to an adherend. The film having a thickness of less than 1 $\mu$m may cause the film to be broken when peeled off from a base material film. The film does not have stiffness, is less handleable in the form of a film laminated with the adhesion-preventing layer, and may make it difficult to perform a step of attaching the laminated film to the water-soluble nonwoven fabric (C). In some cases, the water-soluble film having a thickness of more than 18 $\mu$m brings about the following results: the laminate generates wrinkles when attached to an adherend; after being attached to an adherend, the laminate takes time to be dissolved in water or an aqueous solution, thus decreasing in adherence to an adherend; or when bent, the laminate generates creases, cracks, or breaks, or decreases in planarity.

<Water-soluble Nonwoven Fabric (C)>

A water-soluble nonwoven fabric (C) in the present invention may be constituted by one kind of water-soluble resin, or may be constituted by two or more kinds of water-soluble resins.

The method of producing the nonwoven fabric is not particularly limited. A nonwoven fabric can be produced by forming fleeces using a dry method, wet method, meltblowing method, spun-bonding method, or the like, and binding fibers using a chemical bonding method, thermal bonding method, needlepunching method, water flow interlacing method, or the like.

The thickness of the nonwoven fabric is preferably 80 $\mu$m to 2000 $\mu$m. The nonwoven fabric having a thickness of 80 $\mu$m or more allows the fabric to have excellent roll-conveyability in a spinning machine. In addition, having a thickness of 2000 $\mu$m or less leads to increasing the length of the fabric to be wound up by a spinning machine at a time, thus affording excellent productivity.

In this regard, the above-described thickness refers to the average of 10 values measured as follows: 10 test pieces 2500 mm$^2$ or more in size are taken from a sample in accordance with JIS L1912: 1997; and the thickness of each test piece is measured using a constant-pressure thickness gauge (tradename, PEACOCK (registered trademark), manufactured by Ozaki Mfg. Co., Ltd.; the digital gauge, model PDN-21; the measuring pressure, 0.5 $\pm$ 0.1 kPa; the area of the presser foot, 2500 mm$^2$; and the dial gauge stand, model SIS-6).

The average fiber diameter of the fiber in the nonwoven fabric is preferably 0.001 $\mu$m to 100 $\mu$m from the viewpoints of water-solubility and fiber strength. In addition, the average fiber diameter of the fiber in the nonwoven fabric is more preferably 0.1 $\mu$m or more, still more preferably 1 $\mu$m or more. The nonwoven fabric having an average fiber diameter of 0.001 $\mu$m or more can be made into threads stably during spinning. In addition, the average fiber diameter of the nonwoven fabric is preferably 100 $\mu$m or less, more preferably 50 $\mu$m or less. The nonwoven fabric having an average fiber diameter of 100 $\mu$m or less can provide sufficient flexibility.

The areal weight of the nonwoven fabric is preferably 1 g/m$^2$ to 1000 g/m$^2$. In addition, the areal weight of the nonwoven fabric is more preferably 10 g/m$^2$ or more, still more preferably 15 g/m$^2$ or more. The nonwoven fabric having an areal weight of 1 g/m$^2$ or more makes it possible to enhance the shape stability and dimensional stability of the fibrous structure, and to inhibit the nonwoven fabric from causing processing unevenness and breakage due to elongation when the nonwoven fabric is attached to the water-soluble film (B). In addition, the areal weight of the nonwoven fabric is more preferably 400 g/m$^2$ or less, still more preferably 150 g/m$^2$ or less. The nonwoven fabric having an areal weight of 1000 g/m$^2$ or less is easy to handle when made into roll form, and in addition, makes it possible to inhibit the cushioning characteristics of the nonwoven fabric suitably, to maintain the pressing pressure suitably on the surface of the fibrous structure when the nonwoven fabric is attached to the water-soluble film (B), and thus to perform efficient attaching processing.

The nonwoven fabric may undergo pressing or the like. The pressing may be performed in any step between a step of obtaining a nonwoven fabric and a step after attachment to the water-soluble film (B). To increase the settability during pressing, hot pressing is preferably performed.

In the present invention, both of the water-soluble nonwoven fabric (C) and the water-soluble film (B) contain a water-soluble polymer, and thus, make it possible to produce a laminate having good adhesiveness between the water-soluble nonwoven fabric (C) and the water-soluble film (B). The laminate retains a shape stable to an external force, and has excellent handleability when handled with forceps or by hand.

<Laminate>

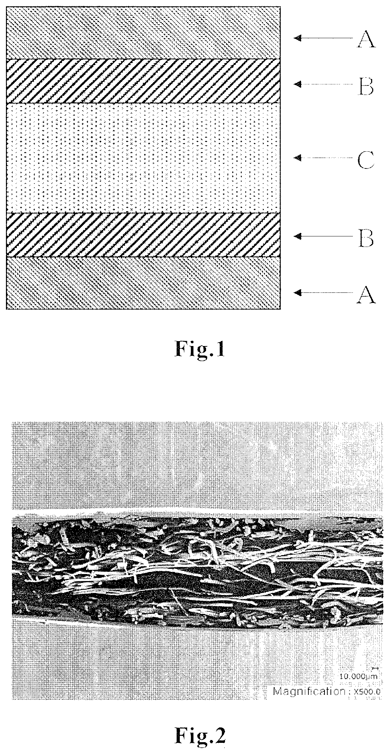

A laminate according to the present invention includes: an adhesion-preventing layer (A) composed of a polylactic acid resin and having a thickness of 10 to 500 nm; a water-soluble film (B) composed of a water-soluble resin and having a thickness of 1 to 18 $\mu$m; and a water-soluble nonwoven fabric (C) composed of a water-soluble resin and having a thickness of 80 to 550 $\mu$m. In addition, this laminate is constituted by the adhesion-preventing layer (A)/the water-soluble film (B)/the water-soluble nonwoven fabric (C)/the water-soluble film (B)/the adhesion-preventing layer (A) in this order from one face of the laminate. FIG. 1 is one example of a schematic diagram of a cross section of a laminate according to the present invention.

In addition, the laminate according to the present invention has a cross section whose scatter diagram satisfies the following Formula 1:

$$0.90 \leq I_{10-45} / I_{max} \leq 0.99 \dots \text{Formula 1}$$

(wherein in Formula 1, $I_{10-45}$ represents an average intensity in the angles of from 10 degrees to 45 degrees, and $I_{max}$ represents the maximum average intensity in 0 to 180 degrees);

the scatter diagram being obtained by:

observing the cross section under a scanning electron microscope at a magnification of 500 times to obtain a digital image;
cutting an image (X) of 720 $\times$ 720 pixels out of the digital image;
binarizing the image (X) with a threshold by a discriminant analysis method to obtain an image (Y), wherein at the threshold the separation metrics is the maximum in the method;
fast-Fourier-transforming the image (Y) to obtain a power spectrum intensity;
converting the power spectrum intensity into decibels; and
then integrating the converted intensity at 5-degree angular intervals from 0 to 180 degrees on the spectrum to obtain the scatter diagram, wherein in the spectrum the central part is assumed as the origin, the positive x axis represents zero degree, and the counterclockwise direction represents a positive angle, and wherein in the scatter diagram the ordinate is an average intensity (I), and the abscissa is an angle.

In addition, a laminate according to the present invention may have the cross section as follows: the image (Y) is obtained by:

observing the cross section of the laminate under the scanning electron microscope at a magnification of 500 times to obtain the digital image;
cutting the image (X) of 720 $\times$ 720 pixels out of the digital image; and
binarizing the image (X) with the threshold by the discriminant analysis method to obtain the image (Y), wherein at the threshold the separation metrics is the maximum in the method; wherein the image (Y) satisfies the following Formula 2:

$$0.30 \leq a / \{a + b\} \leq 0.45 \dots \text{Formula 2}$$

(wherein in Formula 2, "a" represents a photographed area of the portion including the adhesion-preventing layer (A), the water-soluble film (B), and the water-soluble nonwoven fabric (C), and "b" represents a photographed area of the vacant portion surrounded by the adhesion-preventing layer (A), the water-soluble film (B), and the water-soluble nonwoven fabric (C)).

The thickness of a cross section of the laminate according to the present invention is measured using the following measurement method. Specifically, an observation test piece (cross section of a laminate) is produced, using a rotary type microtome (manufactured by Nihon Microtome Laboratory, Inc.) for cut-piece production and figuring. Using a function of a scanning electron microscope (SEM) such as an ultra-deep multi-angle microscope VHX-D500 (manufactured by Keyence Corporation), the magnification is adjusted within the range of from 500 times to 100,000 times so that each layer to be observed can fit between 10% and 90% of the viewing angle. Thus, the thickness of the laminate can be obtained. One example of an image of the laminate according to the present invention, thus obtained and photographed under the scanning electron microscope, is shown in FIG. 2. Here, the thickness of the cross section is defined as the average of the values obtained by measuring the thicknesses of the observation test pieces, in which the observation test pieces are taken from nine points in a rectangular test piece 14.7 cm in length and 12.7 cm in width, in which the nine points are set at 5 cm intervals in the form of a grid parallel in the short-axis direction or the long-axis direction, assuming that the center of the rectangular test piece is the origin. In a case where the rectangular test piece is smaller than the size 14.7 cm in length and 12.7 cm in width, thus not making it possible to take observation test pieces at 5 cm intervals, the observation test pieces may be taken from a plurality of test pieces. The cross section may be parallel in the short-axis direction or parallel in the long-axis direction.

For pretreatment in the thickness measurement method, the surface of the observation test piece is preferably coated with an electroconductive material such as gold, platinum, or carbon, using an ion sputtering device such as "Auto Fine Coater JEC-3000FC" (manufactured by JEOL Ltd.), to prevent electrification due to electrons.

In this regard, in a case where the thickness of each layer is 100 nm or less, thus making it difficult to make observations using the above-described method, observations may be made in the same manner, using a transmission electron microscope (TEM) such as "JEM-2100Plus" (manufactured by JEOL Ltd.) at a magnification of 500,000 times to 1,000,000 times. In a case where it is difficult to make a judgement, it is possible that an image for observation is stored and suitably enlarged (for example, by printing the image in A3 size, using an image zoom, or the like) so that the thickness observed can be calculated.

A method of obtaining a digital image (W) of a cross section from a laminate according to the present invention is not particularly limited. A desired image (at a magnification of 500 times) can be obtained using a method equivalent to the thickness measurement method. To facilitate the below-described fast Fourier transformation, the image is compressed as follows: when the image (X) of $720 \times 720$ pixels is cut out of the digital image (W), the field of view of the digital image (W) is photographed with 1600 pixels in width and 1200 pixels in length so as to correspond to a rectangle 800 $\mu$m in width and 600 $\mu$m in length in actual dimensions; then, the thickness of the laminate is confirmed; in a case where the thickness of the laminate is less than 450 $\mu$m, the image is 80% compressed so that the whole can become 1280 pixels in width and 960 pixels in length; or, in case where the thickness of the laminate is 450 $\mu$m or more, the image is 60% compressed so that the whole can become 960 pixels in width and 720 pixels in length. Out of the image compressed, an image of $720 \times 720$ pixels is cut.

The position of the centroid of the laminate image cut out should be selected in such a manner that the position is the center of the image (X), and in such a manner that two water-soluble films (B) are overall the most parallel with the upper-limit and lower-limit lines in the region of the image (X).

In the present invention, a method to be used to binarize the image (X) is a method of binarization with a threshold at which the separation metrics is the maximum in a discriminant analysis method (what is called Otsu's method). For information processing of an image, commercially available image analysis software such as "ScionImage" (manufactured by Scion Corporation) can be used. In this regard, before the binarization, the portion other than the laminate (outside the laminate) in the image (X) is blackened (masked) so as not to make noise.

A photographed area (a) of the portion including the adhesion-preventing layer (A), the water-soluble film (B), and the water-soluble nonwoven fabric (C) and a photographed area (b) of the vacant portion surrounded by the adhesion-preventing layer (A), the water-soluble film (B), and the water-soluble nonwoven fabric (C) can be obtained by counting the number of pixels of the respective potions, for example, using any kind of image analysis software such as "Analyze Particle".

In this regard, the ratio of each of the photographed area (a) of the portion including the adhesion-preventing layer (A), the water-soluble film (B), and the water-soluble nonwoven fabric (C) and the photographed area (b) of the vacant portion surrounded by the adhesion-preventing layer (A), the water-soluble film (B), and the water-soluble nonwoven fabric (C) means the average of the values obtained by evaluating observation test pieces, in which the test pieces are taken from nine points in a rectangular test piece 14.7 cm in length and 12.7 cm in width, in which the nine points are set at 5 cm intervals in the form of a grid parallel in the short-axis direction or the long-axis direction, assuming that the center of the rectangular test piece is the origin. Here, in a case where the rectangular test piece is smaller than the size 14.7 cm in length and 12.7 cm in width, thus not making it possible to take observation test pieces at 5 cm intervals, the observation test pieces may be taken from a plurality of test pieces. In this regard, the cross section may be parallel in the short-axis direction or parallel in the long-axis direction.

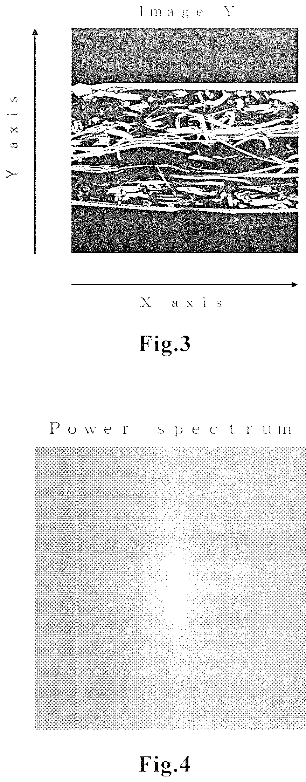

In addition, the laminate according to the present invention is used to analyze an image (Y) by fast Fourier transformation, in which the image (Y) is obtained by binarizing an image (X) with a threshold at which the separation metrics is the maximum in a discriminant analysis method, in which the image (X) is cut to $720 \times 720$ pixels out of a digital image obtained by observing a cross section of the laminate under a scanning electron microscope at a magnification of 500 times. This fast Fourier transformation can be performed, for example, using commercially available image analysis software such as "Image-Pro" (manufactured by Media Cybernetics). In addition, conversion into decibels can be performed using a programming language "C++", "Java" (registered trademark), "Python" (and a plurality of libraries such as OpenCV and NumPy), or the like. FIG. 3 shows one example of an image of a laminate according to the present invention, before the Fourier-transformation. FIG. 4 shows one example of the image in FIG. 3, after the Fourier-transformation.

Or, the above-described series of image analysis (trimming, binarization, Fourier-transformation, and area calculation), a machine language "C++", "Mathematica" (registered trademark), or "Python" (and a plurality of libraries such as "OpenCV", "Numpy", and "Matplotlib") may be used.

In this regard, relevant information on image processing related to a nonwoven fabric alone is partly described in "Microstructural Analysis of Fiber Segments In Nonwoven Fabrics Using SEM and Image Processing" (a magazine "INTERNATIONAL Nonwovens Journal" Volume 10, No. 2, pp. 26-31, Summer, 2001).

A laminate according to the present invention is characterized by a specific structure of a cross section of the laminate, thus can disperse distortion generated by bending, and can have excellent durability in a dry state under a low-humidity environment. Because of this, the laminate is less prone to be damaged by an external force even when packaged in a dry state to be stored for a long time, and is also less prone to be cracked immediately after the package is opened.

In addition, the laminate can be suitably utilized for medical products, such as wound dressing materials and adhesion-preventing materials, and materials for external use for the skin, such as skin care products and adhesive plasters, in medical workplaces, where such products and materials need to be rapidly attached to an adherend. Having both faces formed by an adhesion-preventing layer composed of a polylactic acid resin eliminates the necessity of confirming the front face or the back face when the laminate is attached to an adherend. In addition, the direction in which the laminate is bent makes no difference in durability, and this feature provides easy handling.

<Base Material>

The base material in the below-described section on a method of producing the adhesion-preventing layer (A) and the water-soluble film (B) will be described.

The base material to be used in the present invention is preferably a film composed of a polymeric substance. Examples of the material for the film to be used for the base material (the film is hereinafter referred to as a base material film) include: polyolefins such as polyethylene and polypropylene; polyesters such as polyethylene terephthalate, polybutylene terephthalate, and polyethylene-2,6-naphthalate; polyamides such as nylon 6 and nylon 12; polyvinyl chloride; ethylenevinyl acetate copolymers or saponified products thereof; polystyrene; polycarbonate; polysulfone; polyphenylene oxide; polyphenylene sulfide; aromatic polyamides; polyimides; polyamideimides; cellulose; cellulose acetate; polyvinylidene chloride; polyacrylonitrile; polyvinyl alcohol; and copolymers thereof.

The material for the base material film is preferably a polyester such as polyethylene terephthalate or a polyolefin such as polyethylene or polypropylene from the viewpoint of ensuring adherence between the water-soluble film (B) and the base material film and uniformity in the thickness of each of the water-soluble film (B) and the adhesion-preventing layer (A). A polyester such as polyethylene terephthalate is particularly preferable because of having high wet tension of the surface.

Before the water-soluble film (B) is formed on the base material film using a water-soluble resin, the base material film is more preferably surface-treated with corona discharge, flame, plasma, ultraviolet irradiation, or the like.

The base material film may be any of an unstretched film, a uniaxially-stretched film, or a biaxially-stretched film, and is preferably a biaxially-stretched film from the viewpoints of dimensional stability and mechanical characteristics.

In addition, the base material film may contain various kinds of additives.

The thickness of the base material film is not particularly limited, and is preferably 2 $\mu$m to 1000 $\mu$m, more preferably 10 $\mu$m to 500 $\mu$m, from the viewpoint of economical efficiency.

<Polylactic Acid Resin>

The polylactic acid resin to be used for the laminate according to the present invention refers to a polymer containing poly-D-lactide, poly-L-lactide, or poly-D,L-lactide as a monomer, and is specifically, for example, polylactic acid, poly-L-lactic acid (the L form), poly-D-lactic acid (the D form), poly-DL-lactic acid (the DL form), or the like.

In addition, the weight-average molecular weight of the polylactic acid resin is preferably 10,000 to 400,000, more preferably 20,000 to 300,000, still more preferably 30,000 to 200,000. The weight-average molecular weight of the polylactic acid resin, as used in the present invention, refers to a weight-average molecular weight measured by gel permeation chromatography (hereinafter referred to as GPC) and calculated in terms of polymethyl methacrylate (PMMA), in which GPC a solvent containing sodium trifluoroacetate at 5 mM added to and dissolved in hexafluoroisopropanol (HFIP) is used.

The polylactic acid resin may be mixed with a crystalline homopolylactic acid resin and a noncrystalline homopolylactic acid resin to enhance the solubility in a solvent when a coating liquid for the coating film is produced. In this case, the ratio of the noncrystalline homopolylactic acid resin may be decided on to the extent that the effects of the present invention are not impaired. Additionally, in a case where the layer of a biodegradable material is desired to have relatively high heat resistance, at least one of the polylactic acid resins to be used is preferably a polylactic acid resin having an optical purity of 95% or more.

The polylactic acid resin preferably contains poly-L-lactic acid and/or poly-D-lactic acid as a main component(s). The main component herein refers to a lactic-acid-derived component that accounts for 70 mol% or more and 100 mol% or less with respect to 100 mol% of all the monomer components constituting the polylactic acid resin. The homopolylactic acid resin substantially composed of poly-L-lactic acid and/or poly-D-lactic acid alone are preferably used.

In addition, the amount of poly-D-lactic acid with respect to 100 mol% of the whole polylactic acid resin is preferably 4 mol% to 50 mol%, more preferably 6 mol% to 13 mol%. Containing the poly-D-lactic acid in an amount of 4 mol% or more with respect to 100 mol% of the whole polylactic acid resin results in having suitable solubility in an organic solvent, and thus, the resin is easy to make into a coating agent. Containing the poly-D-lactic acid in an amount of 50 mol% or less with respect to 100 mol% of the whole polylactic acid resin does not adversely affect metabolism, and thus, is preferable.

In addition, the polylactic acid resin may be a copolymerized polylactic acid resin obtained by copolymerizing L-lactic acid and/or D-lactic acid with another monomer component having an ester-forming ability.

Examples of the copolymerizable monomer component include: hydroxycarboxylic acids such as glycolic acid, 3-hydroxybutyric acid, 4-hydroxybutyric acid, 4-hydroxyvaleric acid, and 6-hydroxycaproic acid; compounds containing a plurality of hydroxyl groups in the molecule, such as ethylene glycol, propylene glycol, butanediol, neopentyl glycol, polyethylene glycol, glycerol, polyglycerol, and pentaerythritol, or derivatives of such compounds; compounds containing a plurality of carboxylic groups in the molecule, such as succinic acid, adipic acid, sebacic acid, fumaric acid, terephthalic acid, isophthalic acid, 2,6-naphthalenedicarboxylic acid, 5-sodiumsulfoisophthalic acid, and 5-tetrabutylphosphoniumsulfoisophthalic acid, or derivatives of such compounds; and sugars such as glucose, fructose, xylose, galactose, mannose, erythrose, arabinose, sucrose, maltose lactose, trehalose, and cellobiose.

From these copolymerization components, a component having biodegradability is preferably selected, depending on the usage. These copolymerization components are preferably contained in an amount of 40 mol% or less with respect to 100 mol% of all the monomer components constituting the polylactic acid resin.

A method of producing a polylactic acid resin, as detailed below, include a direct polymerization method to be performed with lactic acid and a ring-opening polymerization method to be performed via lactide.

The polylactic acid resin preferably has a carboxyl-group-end concentration of not more than 30 equivalents/$10^3$ kg, more preferably not more than 20 equivalents/$10^3$ kg, still more preferably not more than 10 equivalents/$10^3$ kg, from the viewpoints of inhibiting a decrease in the strength by hydrolysis, and providing good durability (long-term storability). With the polylactic acid resin having a carboxyl-group-end concentration of not more than 30 equivalents/$10^3$ kg, the concentration of a carboxy group end, which also works as an autocatalyst in hydrolysis, is sufficiently low, can provide practically good durability, and thus, is preferable. The lower limit of the concentration of the carboxyl group end in the polylactic acid resin is not particularly limited, and may be unlimitedly close to 0 equivalent.

Examples of the method of bringing the carboxyl-group-end concentration of the polylactic acid resin to not more than 30 equivalents/$10^3$ kg include: a method in which the concentration is controlled with a catalyst or a heat history during synthesis of the polylactic acid resin; a method in which the heat history is decreased by decreasing the processing temperature or shortening the heating time when the resin is molded into layered form; and a method in which the carboxyl group end of the polylactic acid resin is capped using a reactive compound or the like.

In the method in which the carboxyl group end of the polylactic acid resin is capped using a reactive compound, preferably at least a part of the carboxyl group ends of the polylactic acid resin are capped, more preferably all the ends are capped. Examples of the reactive compound include: condensation-reaction compounds such as aliphatic alcohol and amide compound; addition-reaction compounds such as carbodiimide compounds, epoxy compounds, and oxazoline compounds. From the viewpoint of less proneness to generate a surplus by-product during reaction, addition-reaction compounds are preferable. Among these, carbodiimide compounds are preferable from the viewpoint of efficiency of reaction.

An impact modifier to be used in the present invention to enhance the impact resistance is preferably an aliphatic polyester other than the polylactic acid resin, from the viewpoint of having suitable dispersibility in the polylactic acid resin, and providing a higher effect in a smaller amount.

Specific examples of the aliphatic polyester other than the polylactic acid resin include, but are not particularly limited to, polyglycolic acid, poly(3-hydroxybutyric acid), poly(4-hydroxybutyric acid), poly(4-hydroxyvaleric acid), poly(3-hydroxyhexanoic acid) or polycaprolactone, polyethylene adipate, polyethylene succinate, polybutylene succinate, and polybutylene succinate adipate.

To further enhance the mechanical strength, and maintain the biodegradability, a polybutylene succinate polymer that is an aliphatic polyester other than the polylactic acid resin is preferably used. Polybutylene succinate and polybutylene succinate adipate that have a large effect for enhancing the mechanical strength, and have good compatibility with a polylactic acid resin are more preferable.

The weight-average molecular weight of the polybutylene succinate polymer is preferably 100,000 to 300,000.

The polybutylene succinate polymer is obtained by polycondensing 1,4-butanediol and succinic acid.

The polylactic acid resin can be obtained, for example, by the following method. As raw materials, the lactic acid component which is L-lactic acid or D-lactic acid, and a hydroxycarboxylic acid or the like other than the lactic acid component can be used in combination. Alternatively, a cyclic ester intermediate of a hydroxycarboxylic acid such as lactide or glycolide can be used as a raw material. Furthermore, dicarboxylic acids, glycols, and the like can also be used.

The polylactic acid resin can be obtained using a method of direct dehydration condensation of the above-described raw material such as lactide or glycolide, or a method of ring-opening polymerization of the cyclic ester intermediate. For example, in the case of production by direct dehydration condensation, a high-molecular-weight polymer is obtained through polymerization using the following method; a lactic acid, or a lactic acid and a hydroxycarboxylic acid, is/are azeotropically dehydration-condensed preferably in the presence of an organic solvent, particularly a phenyl ether solvent; water is removed from the solvent distilled preferably through azeotropy; and the resulting substantially anhydrous solvent is returned to the reaction system.

In addition, it is also known that a high-molecular-weight polymer is obtained by ring-opening-polymerizing a cyclic ester intermediate such as lactide under reduced pressure, using a catalyst such as tin octylate. In this case, a polymer containing lactide in a smaller amount can be obtained by using the following method: a method in which an adjustment is made to conditions for removing water and low-molecular-weight compounds during heat refluxing in an organic solvent; a method in which depolymerization reaction is inhibited by deactivating a catalyst after completion of polymerization reaction; a method in which the polymer produced is heat-treated; or the like.

<Water-soluble Resin>

A water-soluble resin to be used in the present invention is a polymer substance that can be dissolved in water, warm water, physiological saline, or an aqueous solution such as a glucose solution. Specific preferable examples include: polyvinyl alcohol or copolymers thereof; polysaccharides such as dextran, agarose, pullulan, chitosan, mannan, carrageenan, alginic acid, starches (oxidized starches, etherified starches, dextrin, and the like), amylose, amylopectin, pectin, lentinan, hyaluronic acid, hylan, and cellulose derivatives (methyl cellulose, ethyl cellulose, carboxymethyl cellulose, carboxyethyl cellulose, hydroxyethyl cellulose, hydroxypropyl cellulose, and the like); polypeptides such as gelatin, collagen, elastin, albumin, hemoglobin, transferrin, globulin, fibrin, fibrinogen, and keratin sulfate; copolymerized polyesters containing a polar group, such as polyvinylpyrrolidone and sulfoisophthalic acid; vinylic polymers such as polyhydroxyethyl methacrylate or copolymers thereof; acrylic polymers; urethane polymers; and ethereal polymers.

In addition, polymers obtained by modifying such a polymer with a functional group such as a carboxyl group, amino group, or methylol group can also be preferably used. Among these, polyvinyl alcohol and pullulan are preferable from the viewpoints of production cost, availability, and hygiene.

The above-described polyvinyl alcohol is a saponified product of polyvinyl acetate, and the degree of saponification is preferably 80 mol% to 99.9 mol%, more preferably 85 mol% to 98 mol%. With the polyvinyl alcohol having a degree of saponification of 99.9 mol% or less, the solubility of the resulting water-soluble nonwoven fabric and water-soluble film (B) in water or an aqueous solution falls within the preferable ranges.

The polyvinyl alcohol in the present invention encompasses a polyvinyl alcohol copolymer. In the polyvinyl alcohol copolymer, the vinyl alcohol unit is preferably 80 mol% to 98 mol%, more preferably 85 mol% to 98 mol%.

The above-described degree of saponification refers to the ratio (mol%) of the number of moles of a vinyl alcohol unit with respect to the total number of moles of the following: a structural unit (typically a vinyl ester unit) that is possessed by polyvinyl alcohol or a copolymer thereof, and can be converted by saponification into the vinyl alcohol unit; and the vinyl alcohol unit. The degree of saponification can be measured in accordance with JIS K6726:1994.

Pullulan is usually advantageous from the viewpoints of availability and cost. Pullulan produced by culturing a yeast such as in the genus *Aureobasidium* in a culture medium containing an amylolytic product is used advantageously. For example, pullulan (manufactured by Hayashibara Co., Ltd.; pullulan in the Japanese Pharmacopoeia) can be suitably used. Without limitation to these, however, another pullulan product can be used, subject to not departing from the object of the present invention. In addition, if desired, maltotriose derivatized, for example, by modification such as esterification at an arbitrary degree of substitution may be the repeat unit.

The weight-average molecular weight of pullulan to be used in the present invention is usually 10,000 to 1,000,000, preferably 50,000 to 500,000. In this regard, the pullulan can be adjusted to a desired rate of dissolution by selecting the weight-average molecular weight or molecular-weight distribution of the pullulan. Although depending on other components to be added, the pullulan having a weight-average molecular weight of 10,000 or more makes the film formation easier, thus being preferable, and 1,000,000 or less enables the dissolution in an aqueous solvent to be preferable.

In the present invention, two or more water-soluble resins may be used in mixture. This makes it possible to obtain a coating film having a high mechanical strength and a re-solubility in an aqueous solution, and additionally having a good adherence to a polylactic acid resin, and thus, is preferable. In this regard, the weight-average molecular weight of the water-soluble resin, as used in the present invention, refers to a weight-average molecular weight measured using GPC, and calculated using a conversion method based on pullulan, maltopentaose, and glucose, in which GPC a solvent containing sodium chloride at 0.1 M added to and dissolved in distilled water is used.

To the extent that the effects of the present invention are not impaired, various additives may be contained in an additive amount of 30 mass% or less with respect to 100 mass% of the whole water-soluble resin. The addition amount of the various additives is not particularly limited to any lower limit, and may be 0 mass% with respect to 100 mass% of the whole.

Examples of the additive that is usable include antioxidants, heat stabilizers, lubricants, nucleating agents, ultraviolet absorbers, X-ray absorbers, and colorants. In addition, to the extent that the effects of the present invention are not impaired, organic or inorganic particles may be contained at 20 mass% or less with respect to 100 mass% of the whole. The additive amount of the additive of any kind is not particularly limited to any lower limit, and may be 0 mass% with respect to 100 mass% of the whole. Examples of the additive that is usable include: particles of calcium carbonate, magnesium oxide, titanium oxide, silicon oxide, calcium fluoride, lithium fluoride, alumina, barium sulfate, zirconia, calcium phosphate, or cross-linked polystyrene; and nanoparticles of a metal such as gold or silver.

<Coating Agent Containing Aliphatic Polyester>

The coating agent containing a polylactic acid resin is preferably a solution containing constituents dissolved uniformly therein. The solvent to be used is preferably, but not particularly limited to, at least a single solvent selected from, or a solution mixture of two or more selected from, the group consisting of butyl alcohol, chloroform, cyclohexane, acetonitrile, dichloromethane, dichloroethane, ethyl acetate, ethyl propionate, ethyl ether, dipropyl ether, and toluene. Dichloromethane and ethyl acetate are particularly preferable from the viewpoints of productivity and handleability.

The solid concentration of the coating agent containing a polylactic acid resin is preferably, but not particularly limited to, 1.0 mass% or more and preferably 10 mass% or less from the viewpoints of productivity, such as the viscosity, drying efficiency, and applicability of the coating agent.

In addition, to obtain applicability, the solution may contain another organic compound as a third component to the extent that the stability of the coating agent containing the polylactic acid resin is maintained.

<Coating Agent Containing Water-soluble Resin>

The coating agent containing a water-soluble resin can be used for an aqueous solution to be used when the water-soluble nonwoven fabric (C) and the water-soluble film (B) are produced, and when the water-soluble nonwoven fabric (C) and the water-soluble film (B) are attached to each other.

The coating agent containing a water-soluble resin is preferably a solution containing constituents dissolved uniformly therein. The solvent to be preferably used is water or a solution mixture of water and lower alcohol. A solution mixture of water and lower alcohol is more preferably used.

In a case where the coating agent containing a water-soluble resin is used for production of the water-soluble film (B), the solid concentration of the coating agent is preferably 1.0 mass% or more and preferably 15 mass% or less from the viewpoints of productivity, such as liquid conveyability, drying efficiency, and applicability. Using a coating agent at a solid concentration higher than 15 mass% may cause the viscosity of the solution to be too high, making it difficult to control the thickness of the water-soluble film (B) in some cases. Using a coating agent at a solid concentration lower than 1.0 mass% may cause incomplete coating due to crawling or the like in some cases.

In addition, to obtain applicability, the solvent mixture may contain another water-soluble organic compound as a third component to the extent that the stability of the coating agent containing the water-soluble resin is maintained. Examples of the water-soluble organic compound include alcohols such as methanol, ethanol, n-propanol, and isopropanol; glycols such as ethylene glycol and propylene glycol; glycol derivatives such as methyl cellosolve, ethyl cellosolve, and n-butyl cellosolve; polyalcohols such as glycerol and waxes; ethers such as dioxane; esters such as ethyl acetate; and ketones such as methylethyl ketone. In addition, the pH of the dispersion solution is preferably 2 to 11 from the viewpoint of the stability of the solution.

In a case where the coating agent is used for the water-soluble nonwoven fabric (C), the solid concentration of the coating agent is preferably 10 mass% or more and preferably 30 mass% or less from the viewpoint of productivity, such as stringiness and drying efficiency. Using the solution having a high solid concentration of more than 30 mass% may cause the viscosity of the solution to be too high, and thus may make the solution difficult to discharge through a spinneret. The solid concentration of less than 10 mass% may cause the ratio of shot generation to be high, causing the appearance to be impaired in some cases. In a case where the coating agent is used for an aqueous solution when the water-soluble nonwoven fabric (C) and the water-soluble film (B) are attached to each other, the solid concentration is preferably 20 mass% or less from the viewpoints of productivity, such as dischargeability, drying efficiency, and attachability. Using the solution having a high solid concentration of more than 20 mass% may cause the viscosity of the solution to be too high, causing the spray nozzle to be clogged in some cases.

<Production Method>

Next, a typical method of producing a laminate according to the present invention will be described.

[Method of Preparing Coating Agent]

The method of preparing a coating agent containing the water-soluble resin and a coating agent containing the polylactic acid resin is not particularly limited. In a case where different kinds of additives such as a cross-linking agent and particles are added to the extent that the effects of the present invention are not impaired, the polymer and the additive are preferably dispersed uniformly in the coating agent. A method that may be used, as required, is, for example: increasing the solubility of the polymer by increasing the temperature of the solvent using a heater or the like; or causing mechanical forced dispersion, using a device such as a homomixer, jet agitator, ball mill, beads mill, kneader, sand mill, or triple roll, in which such a device applies a shearing force or a shearing stress.

[Method of Producing Laminate]

The method that can be used to produce a laminate in the present invention is, for example, but not particularly limited to, the following method.

(1) On the base material film, the water-soluble film (B) composed of the water-soluble resin is produced, on which film the adhesion-preventing layer (A) composed of the polylactic acid resin is formed.
(2) The laminated film formed, in which the water-soluble film (B) and the adhesion-preventing layer (A) are laminated, is mechanically peeled off from the base material film to separate the base material film and the laminated film.
(3) The nonwoven fabric (C) composed of the water-soluble resin is attached in such a manner that the water-soluble film (B) side of the laminated film comes into contact with the water-soluble nonwoven fabric (C). Then, to the other face of the water-soluble nonwoven fabric (C), the water-soluble film (B) side of another laminated film prepared separately from (2) is attached so as to come into contact with the water-soluble nonwoven fabric (C). The method that can be used for adhesion therebetween is, for example, a method in which the surface of the water-soluble film (B) or the water-soluble nonwoven fabric (C) is softened or dissolved with heat, an aqueous solution, or the like, and bonded or fused together.

[Method of Producing Water-soluble Nonwoven Fabric (C)]

The production method is not particularly limited. A dry spinning method is widely applicable in accordance with the kind of the water-soluble resin, and thus, is desirable, in which method a raw spinning solution obtained by dissolving a raw material in a solvent is extruded through a nozzle into heated air to remove the solvent by evaporation.

[Method of Producing Adhesion-preventing Layer (A) and Water-soluble Film (B)]

1. Film-forming Method

Examples of the coating method include, but are not particularly limited to, gravure coating, direct lip coating, slot coating, comma coating, inkjet, and silk-screen printing. Examples of the base material include, but are not particularly limited to, glass plates, metal plates, and plastic films. From the viewpoint of economical efficiency, a plastic film is desirably used as the base material film. In particular, a plastic film having surface smoothness is desirable.
In a case where a biaxially-stretched film of a polyester such as polyethylene terephthalate or a polyolefin such as polypropylene is used as a base material film, the method that may be used is any of the following: off-line coating in which coating is performed after a step of forming a biaxially-stretched film; and in-line coating in which coating is performed in a step of forming a biaxially-stretched film.
In a case where in-line coating is used, coating is preferably performed before the film undergoes heat fixation. Heat fixation refers to crystallizing the stretched film by heat-treating the film retained at a temperature higher than the stretching temperature and lower than the melting point of the film. Accordingly, coating in the longitudinal direction or the transverse direction is preferably performed on an unstretched film, a film that has just been uniaxially stretched, or a film that has just been biaxially stretched. More preferably, coating is performed on a film that has just been uniaxially stretched. Then, still more preferably, the film is further stretched uniaxially or multiaxially, and undergoes heat fixation. A method that can be used to dry a coating film is, for example, a heated roll contacting method, heated medium (air, oil, or the like) contacting method, infrared heating method, and microwave heating method.

In a method of forming a coating film on a base material film by off-line coating, it is preferable, from the viewpoint of the capability to make a thin-film coating at a high speed, that a solution containing coating-film components dispersed in various kind of solvent undergoes gravure coating, reverse coating, spray coating, kiss coating, comma coating, die coating, knife coating, air knife coating, or metaling bar coating. Before being coated, the base material film more preferably undergoes an adhesion-promoting treatment that is, for example, as follows: a corona discharge treatment in air, in nitrogen gas, in a gas mixture of nitrogen and carbon dioxide gas, or in another atmosphere; a plasma treatment under reduced pressure; a flame treatment; or an ultraviolet treatment. Furthermore, the base material film may be anchored, using an anchoring agent such as a urethane polymer, epoxy polymer, or polyethylene imine.

The coating film for obtaining the water-soluble film (B) composed of a water-soluble resin is preferably dried in the range of from 60°C to 180°C in off-line coating, or in the range of from 80°C to 250°C in in-line coating. The drying time is preferably 1 second to 60 seconds, more preferably 3 seconds to 30 seconds.

The coating film for obtaining the adhesion-preventing layer (A) composed of the polylactic acid resin is preferably dried in the range of from 60°C to 110°C in off-line coating, or in the range of from 80°C to 180°C in in-line coating. The drying time is preferably 1 second to 60 seconds. more preferably 3 seconds to 30 seconds.

In this regard, the water-soluble film (B) and the adhesion-preventing layer (A) are desirably laminated in this order on the base material film. The order may be the base material film/the adhesion-preventing layer (A)/the water-soluble film (B), subject to not impairing the peelability of the laminated film from the base material film.

2. Peeling from Base Material Film

Next, the laminated film, in which the water-soluble film (B) and the adhesion-preventing layer (A) are laminated, is mechanically peeled off from the base material film.

3. Fixation of Laminated Film to Support

Furthermore, in order for the laminated film to be easier to handle when attached to the water-soluble nonwoven fabric (C), the laminated film is placed on a support in such a manner to attain the order "the support/the adhesion-preventing layer (A)/the water-soluble film (B)". Examples of the support include, but are not particularly limited to, glass plates, metal plates, and plastic films. From the viewpoint of economical efficiency, a plastic film is preferably used as the support. In particular, a plastic film having surface releasability is preferable.

4. Production of Laminate

Examples of a method of attaching the laminated film to the water-soluble nonwoven fabric (C) include, but are not particularly limited to: a method in which the water-soluble film (B) side of the laminated film or one face of the water-soluble nonwoven fabric (C) is softened with heat, and the side and the face are bonded to each other under pressure; and a method in which the water-soluble film (B) side or one face of the water-soluble nonwoven fabric (C) is dissolved with the water-soluble resin, the side and surface are bonded to each other under pressure to be welded together. Then, the same operation is performed on the other face of the water-soluble nonwoven fabric (C), whereby obtaining a laminate of "the adhesion-preventing layer (A)/the water-soluble film (B)/the water-soluble nonwoven fabric (C)/the water-soluble film (B)/the adhesion-preventing layer (A)".

Examples of the aqueous solution include, but are not particularly limited to, pure water, an aqueous alcohol solution, a mineral dispersion liquid, an aqueous drug dispersion solution, and a water-soluble resin solution (20 mass% or less). Pure water is preferable from an economical viewpoint.

In this regard, the water-soluble resin is softened and dissolved with heat, water, or the like, and firmly attached, as above-described, and hence, the thickness of each layer of the nonwoven fabric and the water-soluble film (B) is smaller after the lamination than before the lamination in some cases.

[Method of Spraying Water or Aqueous Solution]

The method of spraying water or an aqueous solution is not particularly limited. The liquid is dispersed uniformly in fine form in a wide range, using a spraying tool such as a spray or a shower. For example, an accumulator-spray method, nozzle-spray method (with a two-fluid nozzle, three-fluid nozzle, or four-fluid nozzle), or inkjet method can be used. In addition, spray methods are classified into an air-spray method, airless-spray method, and electrostatic method. The air-spray method is based on the principle of atomizing, and in this method, liquid together with compressed air is blown off in mist form from a spray gun. The airless-spray method is a liquid-atomizing method in which liquid itself is pressurized without use of air, and tine water particles can be discharged through a nozzle having micropore sizes. Any of such methods may be used.

The temperature of water or an aqueous solution to be sprayed is not particularly limited, and is preferably 5°C to 35°C from the viewpoint of the shape stability of the fine water particles. For an environment for spraying operation, it is preferable, from the viewpoint of operability, that the temperature is 5°C to 35°C, and that the humidity is 45% RH to 85% RH. Furthermore, it is preferable, from the viewpoint of the shape stability of the fine water particles, that the temperature is 10°C to 25°C, and that the humidity is 45% RH to 65% RH. The average water particle size is preferably 1 to 100 $\mu$m. Here, the average water particle size is the average determined from all the values of measurements made at points, in each of which measurements the particle sizes of 10,000 water particles are measured, using a phase Doppler flowmeter, at a position corresponding to a place just before a place of collision between water particles discharged vertically through the spray nozzle and the nonwoven fabric, in which the particle size is the Sauter mean particle size measured three times at each of the points set at 10 mm intervals in the form of a grid horizontally with the face of attachment, assuming that the vertical line from the nozzle is the center of the grid.

The water attachment density of water or an aqueous solution sprayed on the nonwoven fabric is preferably 1.9 g/m$^2$ or less from the viewpoint of the durability of the laminate in a dry state, and preferably 0.7 g/m$^2$ or more from the viewpoint of adherence between the laminated film and the nonwoven fabric. Furthermore, the density is preferably 1.6 g/m$^2$ or less and 1.0 g/m$^2$ or more from the viewpoint of production efficiency. The water attachment density herein is calculated as follows: four sheets of water-sensitive paper (5 cm square) are placed at a position corresponding to a place just before a place of collision between water particles discharged vertically through the spray nozzle and the nonwoven fabric, in which the sheets are placed about the vertical line from the nozzle horizontally with the face of attachment; water or an aqueous solution is sprayed on the sheets of paper; within 10 seconds after completion of the spraying, the weight of each of the sheets of water-sensitive paper wetted is measured on an electronic balance; the average of the differences between the weights measured and the previously measured weights of the sheets of water-sensitive paper before the spraying is calculated; and the average calculated is divided by the area (5 cm square) of the water-sensitive paper to give the water attachment density. In a case where the spray pattern (wetted mark) is anisotropic, the spray nozzle may be rocked suitably in the horizontal direction with a distance kept constantly between the spray nozzle and the water-sensitive paper.

The time for which water or an aqueous solution is sprayed is preferably 20 seconds or less from the viewpoint of the solubility of the fiber of the nonwoven fabric, more preferably 10 seconds or less, from the viewpoint of production efficiency.

The time from the completion of spraying of water or an aqueous solution to the attachment between the laminated film and the nonwoven fabric is preferably within 30 seconds from the viewpoint of the natural drying of the water-soluble resin, more preferably closer to 0 second from the viewpoint of production efficiency.

[Method of Attachment]

Examples of the method of attaching, to the water-soluble nonwoven fabric (C), the water-soluble film (B) side of the laminated film including the adhesion-preventing layer (A) and the water-soluble film (B) include, but are not particularly limited to: a method in which the fabric and the laminated film are each cut in sheet form, sandwiched between flat plates of metal or the like, and pressed; and a method in which the fabric and the laminated film, in continuous long sheet form, are sandwiched between two rolls, and pressed.

Examples

Examples and Comparative Examples (Examples 1 to 14 and Comparative Examples 1 to 9) of the laminate according to the present invention will be described below. First, methods used to evaluate the characteristics in the present invention are described below.

(1) Measurement of thickness of each constituent (adhesion-preventing layer (A), water-soluble film (B), and water-soluble nonwoven fabric (C)) of laminate

Rectangular test pieces 14.7 cm in length and 12.7 cm in width were placed on a table so as not to be superposed on each other, and left to stand in normal condition for 48 hours or more. Then, observation test pieces were produced with a rotary type microtome (manufactured by Nihon Microtome Laboratory, Inc.) for piece production and figuring. The surface of each piece was coated with gold, using "Auto Fine Coater JEC-3000FC" (manufactured by JEOL Ltd.), at an electric current of 30 mA for a sputtering time of 120 seconds. The normal condition represents a temperature of 20 $\pm$ 2°C and a relative humidity of 65 $\pm$ 4% in accordance with JIS L 0105 5.2 (2006).

Using an ultra-deep multi-angle microscope "VHX-D500" (manufactured by Keyence Corporation) and a transmission electron microscope "JEM-2100Plus" (manufactured by JEOL Ltd.), measurements were made on the cross sections at nine points in the rectangular test piece 14.7 cm in length and 12.7 cm in width, in which the nine points were set at 5 cm intervals in the form of a grid parallel in the short-axis direction or the long-axis direction, assuming that the center of the rectangular test piece is the origin. From the values obtained, the average was determined.

(2) Ratio of photographed area (a) to photographed area (b)

In the same manner as in (1), a cross section of the laminate as an observation test piece was observed using an ultra-deep multi-angle microscope "VHX-D500" (manufactured by Keyence Corporation) at a magnification of 500 times to obtain a digital image (W).

Furthermore, from the image (W), a digital image (X) of $720 \times 720$ pixels was cut out, using a trimming program produced with the programming language "Python". When the image (W) was cut out of the digital image (X), the position of the centroid of the laminate image cut out was adjusted so as to be the center of the image (X) in such a manner that two water-soluble films (B) are overall the most parallel with the upper-limit and lower-limit lines of the region of the digital image (X).

Furthermore, the outside of the laminate was blackened (masked), and then, the above-described image was binarized using an Otsu's binarizing method processing program produced with the programming language "Python".

Furthermore, using an area-calculating program produced with the programming language "Python", the photographed area of the water-soluble nonwoven fabric (C), the water-soluble film (B), and the adhesion-preventing layer (A) and the photographed area of the vacant portion surrounded by (a), the water-soluble nonwoven fabric (C), the water-soluble film (B), and the adhesion-preventing layer (A) were calculated by counting the number of pixels of the respective portions. Specifically, in the image obtained by binarizing the digital image (X), the portion of the white-color pixels (a pixel value of 255 with a 256-step gradation) was regarded as the photographed area of the portion including the adhesion-preventing layer (A), the water-soluble film (B), and the water-soluble nonwoven fabric (C), and the portion of the black-color pixels (a pixel value of 0 with a 256-step gradation) was regarded as the photographed area of the vacant portion surrounded by the adhesion-preventing layer (A), the water-soluble film (B), and the water-soluble nonwoven fabric (C). In addition, from the number of all the pixels of the image (Y), the area of the masked portion, separately calculated, was subtracted to calculate the area {a + b} of the whole laminate containing the vacant portion.

Using the value obtained above, the following ratio ($r_1$) was calculated in accordance with the following Formula 3.

$$r_1 = a \ / \ \{a + b\} \ \ldots \ \text{Formula 3}$$

The value of $r_1$ was rounded to the second decimal place.

The ratio ($r_1$) means the apparent density of the laminate, as determined from the digital image of a cross section of the laminate. With $r_1$ at 0.30 or more, the laminate has sufficient stiffness and excellent handleability. Additionally, with $r_1$ at 0.45 or less, the laminate can absorb a mechanical load generated by bending, thus causing no damage.

(3) Peak position and ratio of intensity ($r_2$) of spectrum

First, on the image (Y) used in (2) above, the direction faced by the water-soluble film (B) was assumed as the x axis, and the direction perpendicular to the x axis was assumed as the y axis. The image (Y) was fast-Fourier-transformed using a calculating program produced with the programming language "Python", and furthermore, the power spectrum intensity was converted into decibels to obtain a spectral image.

Furthermore, on the spectral image obtained as above, the central part was assumed as the origin, the positive x axis was assumed as the zero degree, and the counterclockwise direction was assumed as a positive angle. The intensity was integrated at 5-degree angular intervals from 0 to 180 degrees, and the resulting intensities were averaged to obtain the average intensity (I), which was used to produce a scatter diagram with the angle taken as the abscissa and with the average intensity (I) taken as the ordinate. The position of the peak of the following average intensity (I) was read. Furthermore, the ratio ($r_2$) of the average intensity ($I_{10-45}$) at an angle of 10 to 45 degrees to the maximum average intensity ($I_{max}$) was calculated in accordance with the following Formula 4.

$$r_2 = I_{10-45} \ / \ I_{max} \ \ldots \ \text{Formula 4}$$

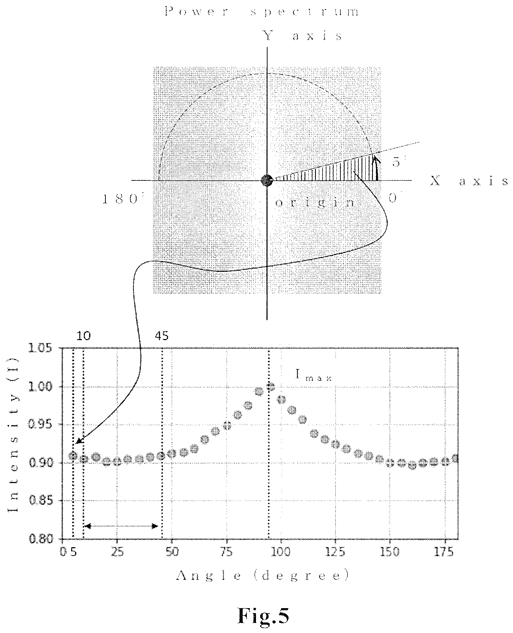

The value of $r_2$ was rounded to the second decimal place. One example of a scatter diagram obtained from this series of positions of the intensity I is shown in FIG. 5.

In the spectrum obtained using the Fourier-transformation of the structural pattern that was formed by the resins constituting the laminate, and obtained with a digital image of a cross section of the laminate, the ratio ($r_2$), on the basis of the angular component exhibiting the strongest characteristic, shows a degree at which the components at 10 degrees to 45 degrees contributed to the structural pattern of the laminate. $r_2$ is preferably 0.90 or more, desirably unlimitedly closer to 1.0. With $r_2$ at 0.90 or more, the laminate can absorb a mechanical load generated by bending, thus being less prone to cause damage.

(4) Durability test

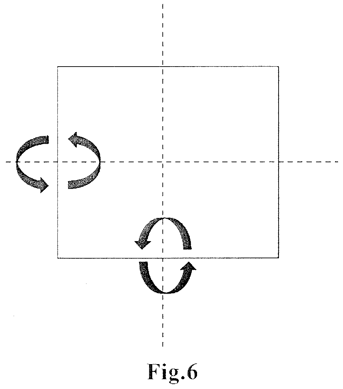

FIG. 6 illustrates a schematic diagram of the method for a durability test on a laminate. In a dry room having a temperature of 20°C and a humidity of 1% RH or less, rectangular test pieces 14.7 cm in length and 12.7 cm in width were placed on a flat table so as not to be superposed on each other, and left to stand for 48 hours or more. Then, in the dry room, the test piece was folded 180 degrees widthwise by hand so as to be halved in area (and width), and then, the test piece was once returned to its original planar form (the first folding). The same test piece was further folded 180 degrees lengthwise by hand in the same direction as in the first folding so as to be halved in area (and width), and then, the test piece was returned to its original planar form (the second folding). Next, the same test piece was folded 180 degrees widthwise in the same manner but in the opposite direction to the direction in the first folding, and then returned to its original planar form (the third folding). Then, the test piece was folded 180 degrees lengthwise, and then returned to its original planar form (the fourth folding).

When each of the second folding and the fourth folding was performed, the test piece was visually observed to verify whether any crack was generated at the crease. A test piece that generated no crack after the fourth folding was rated S; a test piece that generated no crack after the second folding, but generated a crack after the fourth folding was rated A; and a test piece that generated a crack after the second folding was rated B.

(5) Water resistance test

In a laboratory having normal condition, rectangular test pieces 14.7 cm in length and 12.7 cm in width were placed on a flat table so as not to be superposed on each other, and left to stand for 48 hours or more. Then, normal-temperature water (approximately 0.04 ml) measured off using a micropipette was dropped from the tip of the micropipette, the position of which was 10 mm from the upper surface of the test piece, onto nine points set at 5 cm intervals in the form of a grid parallel in the short-axis direction and the long-axis direction in the test piece, assuming that the center of the test piece was the origin. Assuming that the time when the water droplet landed on the test piece was the start time, the test piece was visually checked for any change caused in shape in 15 seconds after the start time. On the basis of whether the laminate was made transparent by dissolution, a test piece that was not transparent 15 seconds later was rated S, a test piece that became transparent within 15 seconds, but was not transparent 5 seconds later was rated A; and a test piece that was at least partly dissolved within less than 5 seconds was rated B.

(6) Adherence test

A rectangular test piece 14.7 cm in length and 12.7 cm in width was cut into squares (3 cm × 3 cm), which were placed in a dish containing pure water. The water-soluble resin was allowed to be dissolved to produce single films composed of the adhesion-preventing layer (A) alone. Pure water in an amount of 100 mg was sprayed on a square polyester film ("LUMIRROR" (registered trademark) #100T60; the size. 5 cm × 5 cm; manufactured by Toray Industries, Inc.), which was thus wetted. To the center of the film, the adhesion-preventing layer (A) previously taken was attached. The film and the layer were pressed for 5 seconds using a dried silicone rubber (the hardness, 20 degrees; the size, 3 cm × 1 cm; manufactured by Kyowa Industrial Co., Ltd.), and firmly attached to each other. Next, the polyester film having the adhesion-preventing layer (A) attached thereto was left to stand vertically for 1 hour or more in an environment having a temperature of 25°C and a relative humidity of 90% in a thermohygrostat (LHU-113, manufactured by ESPEC Corp.) to remove surplus water from the test piece.

Subsequently, the polyester film having the adhesion-preventing layer (A) attached thereto was taken out of the thermohygrostat. The adhesion-preventing layer (A) side was rubbed 10 times with a finger at a force of 800 to 900 gf to check whether such rubbing caused the polyester film and the adhesion-preventing layer (A) to slip from each other. The end of the adhesion-preventing layer (A) or any interference pattern due to being a thin film was visually checked. A test piece that caused no slip was rated A, and a test piece that caused a slip or did not allow verification was rated B.

Each of the materials and the devices used in Examples 1 to 14 and Comparative Examples 1 to 9 will be described below.

[Base material film used]

(Polyester film-1 (hereinafter referred to as "PET-1"):
Biaxially-stretched polyester film ("LUMIRROR" (registered trademark); Type, T60; the thickness, 100 μm; manufactured by Toray Industries, Inc.)

[Polylactic acid resin used]

(Polylactic acid resin-1 (hereinafter referred to as "PLA-1")):
Poly-L-lactic acid-D-lactic acid copolymer (PURASORB (registered trademark) PDL20, manufactured by Corbion N.V.) containing poly-D-lactic acid in an amount of 50 mol% with respect to the whole polymer, and having a weight-average molecular weight of 400,000 in terms of PMMA
(Polylactic acid resin-2 (hereinafter referred to as "PLA-2")):
Poly-L-lactic acid polymer (4060D, manufactured by Nature Works LLC) containing poly-D-lactic acid in an amount of 12 mol% with respect to the whole polymer, and having a weight-average molecular weight of 200,000 in terms of PMMA
(Polylactic acid resin-3 (hereinafter referred to as "PLA-3")):

The following three raw materials were mixed under heating at 110°C in a flask under an argon gas stream.

- L-lactide
  (PURASORB (registered trademark) L, manufactured by Corbion N.V.), 220 g
- Glycolide
  (PURASORB (registered trademark) G, manufactured by Corbion N.V.), 40 g
- Pentaerythritol polyethylene glycol
  (SUNBRIGHT (registered trademark) PTE-10000S; the molecular weight, 10,000; manufactured by NOF Corporation), 60 g

Next, the mixture was heated to 100°C, 0.32 g of tin II ethylhexanoate (manufactured by Fujifilm Wako Pure Chemical Corporation) was added, and the resulting mixture was allowed to react to obtain a block copolymer. The resulting polymer was dissolved in chloroform, washed with diluted hydrochloric acid, and then dropped into a large excess of acetone. A polylactic acid-polyglycolic acid-polyethylene glycol block copolymer (PLA-3) was obtained in the form of a precipitate.
In addition, $^1$H-NMR (the device name, EX-270; the measurement frequency, 400 MHz; the measurement solvent, deuteriochloroform; the number of scans, 8; the measurement temperature, room temperature (approximately 25°C)) was used to evaluate the chemical structure characteristic of each of polyhydroxyalkanoate and polyalkylene glycol, from which chemical structure, the value of integral of the signal of the chemical shift of the protons, the number of hydrogen atoms contained in the repeating units, and the number molecular weight of the repeating monomer were obtained and used to calculate the ratio of the number of moles of lactic acid to 1.0 as the number of moles of glycolic acid, and the ratio was found to be 1:4. In addition, the ratio of the mass of the polyethylene glycol block to the total mass was 6%.
In this regard, the weight-average molecular weight of PLA-3 was 160,000 in terms of PMMA according to GPC. In addition, dichloromethane was used as a solvent when a coating liquid to be used for the film formation of the adhesion-preventing layer (A) was produced.

(Polylactic acid resin-4 (hereinafter referred to as "PLA-4")):

A polylactic acid-polyethylene glycol block copolymer (PLA-4) was obtained in the same manner as in the method of producing PLA-3 except that the raw materials were changed as below.

- DL-lactide:
  (PURASORB (registered trademark) DL, manufactured by Corbion N.V.), 340 g
- 8-arm polyethylene glycol derivative
  (SUNBRIGHT (registered trademark) HGEO-150CS; the molecular weight, 15,000; manufactured by NOF Corporation), 60 g

In this regard, the weight-average molecular weight of PLA-4 was 110,000 in terms of PMMA according to GPC. The ratio of the mass of the polyethylene glycol block to the total mass was 13%. The value obtained by dividing the average molecular weight of the polylactic acid block by 8 as the number of branched chains was 12,000. In addition, dichloromethane was used as a solvent when the coating liquid to be used for the film formation of the adhesion-preventing layer (A) was produced.

[Water-soluble resin used]

(Pullulan-1)

Pullulan (pullulan in the Japanese Pharmacopoeia) having a weight-average molecular weight of approximately 300,000 and a kinematic viscosity of 100 to 180 mm$^2$/second (the measurement conditions for the viscosity. at a temperature of 30°C with an aqueous solution having a solid concentration of 10 mass%).

(Polyvinyl alcohol-1 (hereinafter referred to as "PVA-1"))

Polyvinyl alcohol (JP-10, manufactured by Japan Vam & Poval Co., Ltd.) having a degree of saponification of 88 mol% and a viscosity of 10 mPa·s (an aqueous 4 mass% solution, at 20°C).

(Polyvinyl alcohol-2 (hereinafter referred to as "PVA-2"))

Polyvinyl alcohol (JM-17, manufactured by Japan Vam & Poval Co., Ltd.) having a degree of saponification of 96.5 mol% and a viscosity of 27.5 mPa·s (an aqueous 4 mass% solution, at 20°C).

(Gelatin-1)

Pharmacopoeial purified gelatin (bcMatrix (registered trademark) gelatin HG, manufactured by Nitta Gelatin Inc.)

[Water-soluble nonwoven fabric (C) used]

(Nonwoven fabric-1)

Using Pullulan-1 as a water-soluble resin, fiber threads having an average fiber diameter of 3 μm were obtained through a dry spinning method, and cumulated on a collecting conveyor to produce a nonwoven fabric having a thickness of 370 μm and an areal weight of 32 g/m$^2$. The above-described thickness was obtained by averaging 10 values measured as follows: 10 test pieces 3600 mm$^2$ in size were taken from a sample in accordance with JIS L1912: 1997; and the thickness of each test piece was measured using a constant-pressure thickness gauge (tradename, PEACOCK (registered trademark), manufactured by Ozaki Mfg. Co., Ltd.; the digital gauge, model PDN-21; the measuring pressure, 0.5 ± 0.1 kPa; the area of the presser foot, 2500 mm$^2$; and the dial gauge stand, model SIS-6).
In addition, the areal weight was measured in accordance with JIS L 1096 8.3.2 (1999). Specifically, two 200 mm square test pieces were taken, and the mass (g) per unit area was measured in normal condition. In accordance with the following Formula, the mass per m$^2$ (g/m$^2$) was determined. The average was calculated, and rounded off to the nearest whole number.

$$Sm = W/A$$

Sm: mass per unit area (g/m$^2$) in normal condition
W: mass (g) of test piece in normal condition
A: area (m$^2$) of test piece

In addition, the average fiber diameter was measured as described below. Rectangular test pieces 14.7 cm in length and 12.7 cm in width were placed on a table so as not to be superposed on each other, and left to stand in normal condition for 48 hours or more. Then, the surface of each test piece was coated with gold, using "Auto Fine Coater JEC-3000FC" (manufactured by JEOL Ltd.), at an electric current of 30 mA for a sputtering time of 120 seconds. Next, using an ultra-deep multi-angle microscope VIIX-D500 (manufactured by Keyence Corporation), an image of the surface was photographed at 3000 times at nine points in the rectangular test piece 14.7 cm × 12.7 cm, in which the nine points were set at 5 cm intervals in the form of a grid parallel in the short-axis direction or the long-axis direction, assuming that the center of the test piece was the origin. To the resulting image of each position, the software attached to VHX-D500 was applied; 200 fibers measurable using the planar-point-to-point measurement function were measured per position; and the measurement values were averaged. In this regard, in a case where one angle of view was not sufficient for a total of 200 points, another adjacent angle of view that did not overlap with the former was selected so as to bring the total to 200, and used for measurement.

(Nonwoven fabric-2)

A nonwoven fabric was produced in the same manner as the nonwoven fabric-1 except that the thickness was changed to 1100 $\mu$m, and that the areal weight was changed to 95 g/m$^2$.

(Nonwoven fabric-3)

A nonwoven fabric was produced in the same manner as the nonwoven fabric-1 except that the thickness was changed to 280 $\mu$m, and that the areal weight was changed to 24 g/m$^2$.

(Nonwoven fabric-4)

A nonwoven fabric was produced in the same manner as the nonwoven fabric-1 except that the thickness was changed to 1200 $\mu$m, and that the areal weight was changed to 110 g/m$^2$.

(Nonwoven fabric-5)

A nonwoven fabric was produced in the same manner as the nonwoven fabric-1 except that the thickness was changed to 200 $\mu$m, and that the areal weight was changed to 18 g/m$^2$.

(Nonwoven fabric-6)

A nonwoven fabric was produced in the same manner as the nonwoven fabric-1 except that the thickness was changed to 620 $\mu$m, and that the areal weight was changed to 50 g/m$^2$.

(Nonwoven fabric-7)

A nonwoven fabric was produced in the same manner as the nonwoven fabric-1 except that the thickness was changed to 140 $\mu$m, and that the areal weight was changed to 12 g/m$^2$.

(Nonwoven fabric-8)

A nonwoven fabric was produced in the same manner as the nonwoven fabric-1 except that the thickness was changed to 1700 $\mu$m, and that the areal weight was changed to 150 g/m$^2$.

(Nonwoven fabric-9)

A nonwoven fabric was produced in the same manner as the nonwoven fabric-1 except that the thickness was changed to 1300 $\mu$m, and that the areal weight was changed to 110 g/m$^2$.

(Nonwoven fabric-10)

The water-soluble resin as a material for the nonwoven fabric was changed to PVA-1, and the same method as producing the nonwoven fabric-1 was performed. By this, a nonwoven fabric having a thickness of 370 $\mu$m and an areal weight of 32 g/m$^2$ was produced.

(Nonwoven fabric-11)

The water-soluble resin as a material for the nonwoven fabric was changed to PVA-2, and the same method as producing the nonwoven fabric-1 was performed. By this, a nonwoven fabric having a thickness of 370 $\mu$m and an areal weight of 32 g/m$^2$ was produced.

(Nonwoven fabric-12)

The water-soluble resin as a material for the nonwoven fabric was changed to gelatin-1, and the same method as producing the nonwoven fabric-1 was performed. By this, a nonwoven fabric having a thickness of 1300 $\mu$m and an areal weight of 110 g/m$^2$ was produced.

(Nonwoven fabric-13)

The water-soluble resin as a material for the nonwoven fabric was changed to gelatin-1, and the same method as producing the nonwoven fabric-1 was performed. By this, a nonwoven fabric having a thickness of 1800 $\mu$m and an areal weight of 160 g/m$^2$ was produced.

(Example 1)

Pullulan-1 was used as the water-soluble resin. Specifically, using a heating type homogenizer, Pullulan-1 was dissolved in water to produce an emulsion. One face of a base material film was coated with Pullulan-1 using an applicator method in such a manner that the film would have a thickness of 5 $\mu$m after being dried. The coated film was dried at 90°C in a hot-air drier for 20 seconds to form a water-soluble film (B).

Next, PLA-1 was used as a polylactic acid resin to produce an adhesion-preventing layer (A). Specifically, using a metaling bar, the water-soluble film (B) formed on the base material film was coated with a solution of PLA-1 dissolved in dichloromethane, in such a manner that the film would have a thickness of 200 nm after being dried. The coated film was dried at 80°C in a hot-air drier for 20 seconds to produce a laminated film in which the adhesion-preventing layer (A) composed of a polylactic acid resin and the water-soluble film (B) were laminated. Then, the laminated film was mechanically peeled off from the base material film.

Then, one face of the nonwoven fabric-1 was coated uniformly with pure water at 1.6 g/m$^2$ from a spray nozzle, and then, the nonwoven fabric-1 and the water-soluble film (B) of the laminated film were attached to each other. Then, to the other face of the nonwoven fabric-1, another laminated film was attached in the same manner to produce a laminate in which the laminated film was laminated on both faces of the nonwoven fabric-1.

(Example 2)

A laminate was produced in the same manner as in Example 1 except that the thickness of the adhesion-preventing layer (A) was changed to 300 nm, that the thickness of the water-soluble film (B) was changed to 7 $\mu$m, and that the pure water was sprayed and applied in an amount of 1.2 g/m$^2$ when the laminated film and the water-soluble nonwoven fabric (C) were laminated.

(Example 3)

A laminate was produced in the same manner as in Example 1 except that the thickness of the adhesion-preventing layer (A) was changed to 120 nm, that the thickness of the water-soluble film (B) was changed to 10 $\mu$m, and that the pure water was sprayed and applied in an amount of 1.0 g/m$^2$ when the laminated film and the water-soluble nonwoven fabric (C) were laminated.

(Example 4)

A laminate was produced in the same manner as in Example 1 except that the thickness of the adhesion-preventing layer (A) was changed to 150 nm and that the pure water was sprayed and applied in an amount of 0.8 g/m$^2$ when the laminated film and the water-soluble nonwoven fabric (C) were laminated.

(Example 5)

A laminate was produced in the same manner as in Example 1 except that the thickness of the adhesion-preventing layer (A) was changed to 50 nm, that the thickness of the water-soluble film (B) was changed to 18 $\mu$m, that the water-soluble nonwoven fabric (C) was changed to the nonwoven fabric-2, and that the pure water was sprayed and applied in an amount of 1.4 g/m$^2$ when the laminated film and the water-soluble nonwoven fabric (C) were laminated.

(Example 6)

A laminate was produced in the same manner as in Example 1 except that the thickness of the adhesion-preventing layer (A) was changed to 150 nm, that the thickness of the water-soluble film (B) was changed to 12 $\mu$m, that the water-soluble nonwoven fabric (C) was changed to the nonwoven fabric-3, and that the pure water was sprayed and applied in an amount of 1.0 g/m$^2$ when the laminated film and the water-soluble nonwoven fabric (C) were laminated.

(Example 7)

A laminate was produced in the same manner as in Example 1 except that the thickness of the adhesion-preventing layer (A) was changed to 100 nm, that the water-soluble nonwoven fabric (C) was changed to the nonwoven fabric-4, and that the pure water was sprayed and applied in an amount of 1.0 g/m$^2$ when the laminated film and the water-soluble nonwoven fabric (C) were laminated.

(Example 8)

A laminate was produced in the same manner as in Example 1 except that the thickness of the adhesion-preventing layer (A) was changed to 500 nm, that the thickness of the water-soluble film (B) was changed to 2 $\mu$m, that the water-soluble nonwoven fabric (C) was changed to the nonwoven fabric-2, and that the pure water was sprayed and applied in an amount of 1.4 g/m$^2$ when the laminated film and the water-soluble nonwoven fabric (C) were laminated.

(Example 9)

A laminate was produced in the same manner as in Example 1 except that the thickness of the adhesion-preventing layer (A) was changed to 500 nm, that the thickness of the water-soluble film (B) was changed to 8 $\mu$m, that the water-soluble nonwoven fabric (C) was changed to the nonwoven fabric-5, and that the pure water was sprayed and applied in an amount of 0.7 g/m$^2$ when the laminated film and the water-soluble nonwoven fabric (C) were laminated.

(Example 10)

A laminate was produced in the same manner as in Example 1 except that the thickness of the adhesion-preventing layer (A) was changed to 300 nm, that the resin used for the water-soluble film (B) was changed to PVA-1, that the thickness of the water-soluble film (B) was changed to 7 $\mu$m, that the water-soluble nonwoven fabric (C) was changed to the nonwoven fabric-10, and that the pure water was sprayed and applied in an amount of 1.2 g/m$^2$ when the laminated film and the water-soluble nonwoven fabric (C) were laminated.

(Example 11)

A laminate was produced in the same manner as in Example 1 except that the thickness of the adhesion-preventing layer (A) was changed to 120 nm, that the resin used for the water-soluble film (B) was changed to PVA-1, that the thickness of the water-soluble film (B) was changed to 10 $\mu$m, that the water-soluble nonwoven fabric (C) was changed to the nonwoven fabric-10, and that the pure water was sprayed and applied in an amount of 1.0 g/m$^2$ when the laminated film and the water-soluble nonwoven fabric (C) were laminated.

(Example 12)

A laminate was produced in the same manner as in Example 1 except that the resin used for the water-soluble film (B) was changed to PVA-2 and that the water-soluble nonwoven fabric (C) was changed to the nonwoven fabric-11.

(Example 13)

A laminate was produced in the same manner as in Example 1 except that the thickness of the adhesion-preventing layer (A) was changed to 400 nm, that the resin used for the water-soluble film (B) was changed to gelatin-1, that the thickness of the water-soluble film (B) was changed to 18 $\mu$m, that the water-soluble nonwoven fabric (C) was changed to the nonwoven fabric-12, and that the pure water was sprayed and applied in an amount of 1.4 g/m$^2$ when the laminated film and the water-soluble nonwoven fabric (C) were laminated.

(Example 14)

A laminate was produced in the same manner as in Example 1 except that the thickness of the adhesion-preventing layer (A) was changed to 180 nm, that the resin used for the water-soluble film (B) was changed to gelatin-1, that the thickness of the water-soluble film (B) was changed to 16 μm, that the water-soluble nonwoven fabric (C) was changed to the nonwoven fabric-13, and that the pure water was sprayed and applied in an amount of 1.4 g/m² when the laminated film and the water-soluble nonwoven fabric (C) were laminated.

(Comparative Example 1)

A laminate was produced in the same manner as in Example 1 except that the thickness of the adhesion-preventing layer (A) was changed to 150 nm and that the pure water was sprayed and applied in an amount of 2.4 g/m² when the laminated film and the water-soluble nonwoven fabric (C) were laminated.

(Comparative Example 2)

A laminate was produced in the same manner as in Example 1 except that the thickness of the adhesion-preventing layer (A) was changed to 150 nm and that the pure water was sprayed and applied in an amount of 2.0 g/m² when the laminated film and the water-soluble nonwoven fabric (C) were laminated.

(Comparative Example 3)

A laminate was produced in the same manner as in Example 1 except that the thickness of the adhesion-preventing layer (A) was changed to 150 nm, that the water-soluble nonwoven fabric (C) was changed to the nonwoven fabric-6, and that the pure water was sprayed and applied in an amount of 0.7 g/m² when the laminated film and the water-soluble nonwoven fabric (C) were laminated.

(Comparative Example 4)

A laminate was produced in the same manner as in Example 1 except that the thickness of the adhesion-preventing layer (A) was changed to 150 nm, that the thickness of the water-soluble film (B) was changed to 23 μm, and that the water-soluble nonwoven fabric (C) was changed to the nonwoven fabric-2.

(Comparative Example 5)

A laminate was produced in the same manner as in Example 1 except that the thickness of the adhesion-preventing layer (A) was changed to 150 nm, that the water-soluble nonwoven fabric (C) was changed to the nonwoven fabric-7, and that the pure water was sprayed and applied in an amount of 2.0 g/m² when the laminated film and the water-soluble nonwoven fabric (C) were laminated.

(Comparative Example 6)

A laminate was produced in the same manner as in Example 1 except that the thickness of the adhesion-preventing layer (A) was changed to 150 nm, that the water-soluble nonwoven fabric (C) was changed to the nonwoven fabric-2, that the pure water was sprayed and applied in an amount of 1.0 g/m² when the laminated film and the water-soluble nonwoven fabric (C) were laminated, and that only one face of the water-soluble nonwoven fabric (C) was attached to the laminated film, to the other face of which water-soluble nonwoven fabric (C) the pure water was sprayed and applied in an amount of 1.0 g/m², and the water-soluble film (B) alone was attached, whereby the layered structure became A/B/C/B.

(Comparative Example 7)

A laminate was produced in the same manner as in Example 1 except that the thickness of the adhesion-preventing layer (A) was changed to 9 nm, that the water-soluble nonwoven fabric (C) was changed to the nonwoven fabric-8, and that the pure water sprayed was applied in an amount of 1.8 g/m² when the laminated film and the water-soluble nonwoven fabric (C) were laminated.

(Comparative Example 8)

A laminate was produced in the same manner as in Example 1 except that the thickness of the adhesion-preventing layer (A) was changed to 1500 nm, that the thickness of the water-soluble film (B) was changed to 1.5 $\mu$m, that the water-soluble nonwoven fabric (C) was changed to the nonwoven fabric-9, and that the pure water was sprayed and applied in an amount of 1.8 g/m$^2$ when the laminated film and the water-soluble nonwoven fabric (C) were laminated.

(Comparative Example 9)

A laminate was produced in the same manner as in Example 1 except that the thickness of the adhesion-preventing layer (A) was changed to 150 nm. that the water-soluble nonwoven fabric (C) was changed to the nonwoven fabric-10, that the resin used for the water-soluble film (B) was changed to PVA-1, and that the pure water was sprayed and applied in an amount of 2.4 g/m$^2$ when the laminated film and the water-soluble nonwoven fabric (C) were laminated.

The results of the characteristics of Examples 1 to 14 are shown in Table 1, and the results of the characteristics of Comparative Examples 1 to 9 are shown in Table 2.

[Table 1]

| Item | | | Example 1 | Example 2 | Example 3 | Example 4 | Example 5 | Example 6 | Example 7 |
|---|---|---|---|---|---|---|---|---|---|
| Type of water-soluble resin | | | Pullulan-1 | Pullulan-1 | Pullulan-1 | Pullulan-1 | Pullulan-1 | Pullulan-1 | Pullulan-1 |
| Layered structure | | | A/B/C/B/A | A/B/C/B/A | A/B/C/B/A | A/B/C/B/A | A/B/C/B/A | A/B/C/B/A | A/B/C/B/A |
| Thickness of each layer in laminate | (A) | nm | 200 | 300 | 120 | 150 | 50 | 150 | 100 |
| | (B) | μm | 5 | 7 | 10 | 5 | 18 | 12 | 5 |
| | (C) | μm | 110 | 125 | 133 | 140 | 350 | 100 | 450 |
| Amount of water for attachment | | g/m² | 1.6 | 1.2 | 1.0 | 0.8 | 1.4 | 1.0 | 1.0 |
| Ratio of area (a) | ($r_1$) | - | 0.45 | 0.35 | 0.30 | 0.30 | 0.40 | 0.45 | 0.32 |
| Power spectrum | Position of peak | degrees | 95 | 95 | 95 | 95 | 90 | 95 | 95 |
| | Ratio of intensity ($r_2$) | - | 0.90-0.91 | 0.91-0.92 | 0.92-0.93 | 0.93-0.94 | 0.91-0.92 | 0.90-0.91 | 0.92-0.93 |
| Evaluation | Durability | | A | S | S | S | A | S | A |
| | Water resistance | | S | S | A | S | A | S | A |
| | Adherence | | A | A | A | A | A | A | A |
| Item | | | Example 8 | Example 9 | Example 10 | Example 11 | Example 12 | Example 13 | Example 14 |
| Type of water-soluble resin | | | Pullulan-1 | Pullulan-1 | PVA-1 | PVA-1 | PVA-2 | Gelatin-1 | Gelatin-1 |
| Layered structure | | | A/B/C/B/A | A/B/C/B/A | A/B/C/B/A | A/B/C/B/A | A/B/C/B/A | A/B/C/B/A | A/B/C/B/A |
| Thickness of each layer in laminate | (A) | nm | 500 | 500 | 300 | 120 | 200 | 400 | 180 |
| | (B) | μm | 2 | 8 | 7 | 10 | 5 | 18 | 16 |
| | (C) | μm | 350 | 80 | 125 | 133 | 110 | 350 | 520 |
| Amount of water for attachment | | g/m² | 1.4 | 0.7 | 1.2 | 1.0 | 1.6 | 1.4 | 1.4 |
| Ratio of area (a) | ($r_1$) | - | 0.40 | 0.43 | 0.35 | 0.30 | 0.45 | 0.40 | 0.33 |
| Power spectrum | Position of peak | degrees | 95 | | 95 | 95 | 95 | 90 | 95 |
| | Ratio of intensity ($r_2$) | - | 0.91-0.92 | 0.90-0.91 | 0·91-0.92 | 0.92-0.93 | 0.90-0.91 | 0.91-0.92 | 0,92-0.93 |
| Evaluation | Durability | | A | A | S | S | A | A | A |
| | Water resistance | | S | S | S | A | S | S | S |
| | Adherence | | A | A | A | A | A | A | A |

EP 4 563 346 A1

24

[Table 2]

| Item | | | Comparative Example 1 | Comparative Example 2 | Comparative Example 3 | Comparative Example 4 | Comparative Example 5 |
|---|---|---|---|---|---|---|---|
| Type of water-soluble resin | | | Pullulan-1 | Pullulan-1 | Pullulan-1 | Pullulan-1 | Pullulan-1 |
| Layered structure | | | A/B/C/B/A | A/B/C/B/A | A/B/C/B/A | A/B/C/B/A | A/B/C/B/A |
| Thickness of each layer in laminate | (A) | nm | 150 | 150 | 150 | 150 | 150 |
| | (B) | μm | 5 | 5 | 5 | 23 | 5 |
| | (C) | μm | 70 | 80 | 240 | 350 | 30 |
| Amount of water for attachment | | g/m2 | 2.4 | 2.0 | 0.7 | 1.6 | 2.0 |
| Ratio of area (a) | ($r_1$) | - | 0.65 | 0.55 | 0.28 | 0.49 | 0.45 |
| Power spectrum | Position of peak | degrees | 95 | 95 | - | 90 | 95 |
| | Ratio of intensity ($r_2$) | - | 0.86-0.87 | 0.89-0.90 | - | 0.91-0.92 | 0.86-0.88 |
| Evaluation | Durability | | B | B | B | B | B |
| | Water resistance | | A | A | A | A | A |
| | Adherence | | A | A | A | A | A |
| Item | | | Comparative Example 6 | Comparative Example 7 | Comparative Example 8 | Comparative Example 9 | |
| Type of water-soluble resin | | | Pullulan-1 | Pullulan-1 | Pullulan-1 | PVA-1 | |
| Layered structure | | | A/B/C/B/ | A/B/C/B/A | A/B/C/B/A | A/B/C/B/A | |
| Thickness of each layer in laminate | (A) | nm | 150 | 9 | 1500 | 150 | |
| | (B) | μm | 5 | 5 | 1.5 | 5 | |
| | (C) | μm | 350 | 470 | 350 | 70 | |
| Amount of water for attachment | | g/m2 | 1.0 | 1.8 | 1.8 | 2.4 | |
| Ratio of area (a) | ($r_1$) | - | 0.74 | 0.60 | 0.40 | 0.65 | |
| Power spectrum | Position of peak | degrees | 95 | 95 | 85. 95 | 95 | |
| | Ratio of intensity ($r_2$) | - | 0.89-0.90 | 0.90 | 0.88-0.89 | 0.86-0.87 | |
| Evaluation | Durability | | B | B | B | B | |
| | Water resistance | | B | B | A | A | |
| | Adherence | | A | A | B | A | |

As shown in Table 1, the evaluations of the durability, water resistance, and adherence in Examples 1 to 14 were good. On the other hand, Comparative Examples 1 to 9 each scored a poor point about at least one of the items, as shown in Table 2.

Specifically, $r_1$ and $r_2$ characteristic of the structure of the laminate did not satisfy the scope of the invention in Comparative Examples, in which the laminates failed to absorb a mechanical load generated by folding, thus causing damage. Hence, the durability was rated B.

Additionally, in Comparative Example 3, the water-soluble film (B) and the water-soluble nonwoven fabric (C) had insufficient adherence therebetween, and were easily peeled off from each other, not making it possible to stably perform durability testing and observation of a cross section of the laminate.

Additionally, in Comparative Example 8, the adhesion-preventing layer (A) had a thickness of 1500 nm, which was too thick. Thus, the adhesive force was insufficient, causing the layer to easily slip off the adherend. Hence, the adherence was rated B.

In Comparative Example 6, the water-soluble film (B) was not covered with the adhesion-preventing layer (A). In Comparative Example 7, the adhesion-preventing layer (A) did not have a sufficient thickness. Accordingly, the film and the layer were dissolved instantly when brought into contact with water droplets. Hence, the water resistance was rated B.

Industrial Applicability

A laminate according to the present invention can be suitably used particularly for medical products, such as wound dressing materials and adhesion-preventing materials, and for materials for external use for the skin, such as skin care products and adhesive plasters.

## Claims

1.    A laminate comprising: an adhesion-preventing layer (A) composed of a polylactic acid resin and having a thickness of 10 to 500 nm;

a water-soluble film (B) composed of a water-soluble resin and having a thickness of 1 to 18 $\mu$m; and
a water-soluble nonwoven fabric (C) composed of a water-soluble resin and having a thickness of 80 to 550 $\mu$m;
wherein the adhesion-preventing layer (A), the water-soluble film (B), and the water-soluble nonwoven fabric (C) are laminated in the order (A)/(B)/(C)/(B)/(A);
wherein the laminate has a cross section of which scatter diagram satisfies the following Formula 1:

$$0.90 \leq I_{10\text{-}45} / I_{max} \leq 0.99 \ldots \text{Formula 1}$$

(wherein in Formula 1, $I_{10\text{-}45}$ represents an average intensity in the angles of from 10 to 45 degrees, and $I_{max}$ represents the maximum average intensity in 0 to 180 degrees);
said scatter diagram being obtained by: observing the cross section under a scanning electron microscope at a magnification of 500 times to obtain a digital image; cutting an image (X) of 720 × 720 pixels out of the digital image; binarizing the image (X) with a threshold by a discriminant analysis method to obtain an image (Y), wherein at the threshold the separation metrics is the maximum in the method; fast-Fourier-transforming the image (Y) to obtain a power spectrum intensity; converting the power spectrum intensity into decibels; and then integrating the converted intensity at 5-degree angular intervals from 0 to 180 degrees on the spectrum to obtain the scatter diagram, wherein in the spectrum the central part is assumed as the origin, the positive x axis represents zero degree, and the counterclockwise direction represents a positive angle, and wherein in the scatter diagram the ordinate is an average intensity (I), and the abscissa is an angle.

2.    The laminate according to claim 1,
wherein the image (Y) is obtained by:

observing the cross section under the scanning electron microscope at a magnification of 500 times to obtain the digital image;
cutting the image (X) of 720 × 720 pixels out of the digital image; and
binarizing the image (X) with the threshold by the discriminant analysis method to obtain the image (Y), wherein at the threshold the separation metrics is the maximum in the method; and
wherein the image (Y) satisfies the following Formula 2:

$$0.30 \leq a / \{a + b\} \leq 0.45 \ldots \text{Formula 2}$$

(wherein in Formula 2, "a" represents a photographed area of the portion including the adhesion-preventing layer (A), the water-soluble film (B), and the water-soluble nonwoven fabric (C), and "b" represents a photographed area of the vacant portion surrounded by the adhesion-preventing layer (A), the water-soluble film (B), and the water-soluble nonwoven fabric (C)).

3.  The laminate according to claim 1 or 2, wherein the water-soluble resin is pullulan.

Fig.1

Fig.2

Fig.3

Fig.4

Fig.5

Fig.6

## INTERNATIONAL SEARCH REPORT

| International application No. |
|---|
| **PCT/JP2023/027115** |

### A. CLASSIFICATION OF SUBJECT MATTER

***B32B 27/12***(2006.01)i; ***A61L 15/26***(2006.01)i; ***A61L 15/28***(2006.01)i; ***A61L 15/42***(2006.01)i; ***A61P 17/02***(2006.01)i; ***B32B 5/02***(2006.01)i; ***B32B 27/36***(2006.01)i

FI: B32B27/12; A61L15/26 100; A61L15/28 100; A61L15/42 100; A61P17/02; B32B5/02 Z; B32B27/36

According to International Patent Classification (IPC) or to both national classification and IPC

### B. FIELDS SEARCHED

Minimum documentation searched (classification system followed by classification symbols)

B32B27/12; A61L15/26; A61L15/28; A61L15/42; A61P17/02; B32B5/02; B32B27/36

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | WO 2015/152204 A1 (TORAY INDUSTRIES) 08 October 2015 (2015-10-08) claims, paragraphs [0011], [0035]-[0042], [0053], [0093]-[0106], examples, fig. 2 | 1-3 |
| A | JP 2012-95731 A (GUNZE LTD) 24 May 2012 (2012-05-24) claims, paragraphs [0007], [0018]-[0025], example 9. fig. 1 | 1-3 |
| A | WO 2018/062464 A1 (TORAY INDUSTRIES) 05 April 2018 (2018-04-05) claims, paragraphs [0050]-[0053] | 1-3 |
| A | JP 2022-523952 A (BAER, Hans Ulrich) 27 April 2022 (2022-04-27) claims, paragraphs [0025]-[0034], fig. 1 | 1-3 |
| A | JP 2015-16613 A (HITACHI CHEMICAL CO LTD) 29 January 2015 (2015-01-29) claims, paragraphs [0002], [0027]-[0032], fig. 1 | 1-3 |

☐ Further documents are listed in the continuation of Box C.  ☑ See patent family annex.

| | |
|---|---|
| \* Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **12 October 2023** | **24 October 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT
### Information on patent family members

International application No.

**PCT/JP2023/027115**

| Patent document cited in search report | | | Publication date (day/month/year) | Patent family member(s) | | | Publication date (day/month/year) |
|---|---|---|---|---|---|---|---|
| WO | 2015/152204 | A1 | 08 October 2015 | US | 2017/0136157 | A1 | |
| | | | | claims, paragraphs [0011], [0035]-[0042], [0053], [0093]-[0106], examples, fig. 2 | | | |
| | | | | EP | 3132927 | A1 | |
| | | | | CA | 2940435 | A | |
| | | | | KR | 10-2016-0138379 | A | |
| | | | | CN | 106457750 | A | |
| JP | 2012-95731 | A | 24 May 2012 | (Family: none) | | | |
| WO | 2018/062464 | A1 | 05 April 2018 | US | 2019/0216971 | A1 | |
| | | | | claims, paragraphs [0030]-[0033] | | | |
| | | | | EP | 3520825 | A1 | |
| | | | | CA | 3038959 | A | |
| | | | | CN | 109715222 | A | |
| | | | | KR | 10-2019-0065240 | A | |
| | | | | RU | 2019113140 | A | |
| | | | | CN | 114681683 | A | |
| JP | 2022-523952 | A | 27 April 2022 | US | 2022/0133959 | A1 | |
| | | | | claims, paragraphs [0027]-[0038], fig. 1 | | | |
| | | | | WO | 2020/178271 | A1 | |
| | | | | EP | 3705142 | A1 | |
| | | | | AU | 2020231037 | A | |
| | | | | ES | 2941614 | T | |
| JP | 2015-16613 | A | 29 January 2015 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6119863 B

- JP 7077621 B

**Non-patent literature cited in the description**

- Microstructural Analysis of Fiber Segments In Nonwoven Fabrics Using SEM and Image Processing. INTERNATIONAL Nonwovens Journal. Summer, 2001, vol. 10, 26-31